# EUROPEAN PATENT APPLICATION

(11) **EP 2 075 331 A1**
(43) Date of publication of application: **01.07.2009**
(21) Application number: 07150478.1
(22) Date of filing: 28.12.2007
(51) Int. Cl.: C12N 9/96, C12N 9/12, C12Q 1/68

(54) **Modified enzymes and their uses**

(71) Applicant: QIAGEN GmbH, 40724 Hilden (DE)
(72) Inventor: Peters, Lars-Erik, Lafayette Colorado 80026 (US); Fang, Nan, 41468 Neuss (DE); Cramer, Janina, 40723 Hilden (DE)
(74) Representative: Vossius & Partner

(57) **Abstract**

The invention relates to an active enzyme of bacterial, fungal, viral, or archae origin, wherein the enzyme is coupled with at least one polymer having a molecular weight between about 500 to about 20,000 daltons selected from the group consisting of polyethylene glycol and polypropylene glycol. The invention relates in particular to uses thereof of such as in molecular biology as well as kits comprising such enzymes. In preferred embodiments the enzymes of the invention are nucleic acid modifying or replicating enzymes.

## Description

### FIELD OF THE INVENTION

The invention is in the field of molecular biology, diagnostics, and in *in-vitro* assays. In particular the invention is in the field of enzyme chemistry, more in particular the field of storage and activity of enzymes as well as in particular DNA modifying and amplification enzymes.

### BACKGROUND OF THE INVENTION

Enzymes are proteins that catalyze chemical reactions. Almost all processes in a biological cell need enzymes in order to occur at significant rates. Like all catalysts, enzymes work by lowering the activation energy for a reaction, thus dramatically accelerating the rate of the reaction. As with all catalysts, enzymes are not consumed by the reactions they catalyze, nor do they alter the equilibrium of these reactions. However, enzymes do differ from most other catalysts by being much more specific. Enzymes are known to catalyze about 4,000 biochemical reactions.
Enzyme activity can be affected by other molecules. Inhibitors are molecules that decrease enzyme activity; activators are molecules that increase activity. Many drugs and poisons are enzyme inhibitors. Activity is also affected by temperature, chemical environment (e.g. pH), and the concentration of substrate. Many enzymes are used commercially, for example, in the synthesis of antibiotics. In addition, some household products use enzymes to speed up biochemical reactions (e.g., enzymes in biological washing powders break down protein or fat stains on clothes; enzymes in meat tenderizers break down proteins, making the meat easier to chew).
The stability of peptide bonds ensures that the primary structure of proteins remain intact under biological conditions. Of course, proteases in the environment can cleave the bonds and break a protein apart. Long term chemical changes to proteins include deamidation of asparagine (alkaline pH and phosphates in buffers promote this), oxidation of thiol- or aromatic ring-containing amino acids (usually by heavy metal ions), beta elimination, etc.

The tertiary structures of proteins which largely determine the protein's function are vulnerable to changes in their environment. The chances of adversely affecting proteins increase as they are manipulated more (purification, freezing, thawing). Proteins at 4°C usually have shelf-lives of only months.

The first hint that a protein solution has stability problems is the appearance of precipitates in the protein solution. Such precipitation can occur over long term even if precautions have been taken to avoid acute stresses (like freezing and thawing).

One can wear gloves and use clean tubes and pipet tips, to protect protein solutions from contamination with proteases and chemicals in the environment.

Proteins need to be stored at a high level of concentration - at least 1 mg/ml. This may not be realistic in some cases and so, if possible, an inert protein such as BSA should be added to raise the total protein concentration to 10-15 mg/ml. The draw back of course is that BSA may interfere with some reactions and applications.

Protein solutions should not be vigorously shaken by vortexing. Shaking can not only generates bubbles and raises oxygen levels in the solution, but can also affect protein structure because of the high shearing forces. Many proteins so far require detergents for storage. Shaking such a solution will result in bubble production.

The range of temperatures that a protein goes through during freezing and thawing exposes it to extremes of pH and salt. To lower this degrading effect during freezing, protein solutions should be snap frozen by immersing tubes in dry ice and ethanol/acetone mixture.
One needs to be careful about the buffer used to store a protein in. For enzymes, storage buffers ideally are the same ones in which the enzymes show good activity. Similarly for pH although it may need to be different if the optimal pH is unsuitable for downstream applications like chromatography. Phosphates in some buffers can inhibit certain enzymes. Some proteins require certain ions like magnesium and/or certain concentration of NaCl for optimal stability. At the same time even 150 mM NaCl can promote aggregation of certain proteins. Reducing environment, because of low pH and reducing agents is usually detrimental to proteins that have disulfide bonds (usually lumenal and extracellular proteins). However, they are usually needed (1-5 mM) for proteins that have free thiol groups, usually intracellular proteins. Some proteins can also be more stable when their ligands are present in the solution. E.g., galectin proteins retain functionality when stored with lactose.

Heavy metal ions can be damaging to many proteins, primarily by oxidizing thiol groups. Reducing agents like DTT and/or 0.1 mM EDTA can help in preventing such damage.

Lyophilization is a useful process for long term storage. However, the protein sample needs to be rapidly frozen before lyophilizing.

Some studies have indicated that 50 mM each of arginine and glutamic acid in solution significantly increases protein stability (and solubility) without adversely affecting protein activity.

Azide (sodium) in the storage solution or thiomerosal can prevent microbial contamination of the solution which is important for 4°C storage.

Protein solutions can also be stored as 'salted-out' with ammonium sulfate (usually 70 % saturated). Even at 4°C, such salt-outs are stable for many months. The salt can be removed by dialysis.

Citrate, tris and histidine buffers are less likely to undergo pH changes during freezing and thawing. Sodium phosphate buffers however undergo big changes.

Numerous enzyme applications require certain conditions which are not suited for enzyme activity or storage. In DNA amplification for example there are a number of applications that require a detergent-free reaction buffer system. PCR products used for DHPLC analysis are recommended to be free of detergents. Detergents can cause foaming when PCR products are spotted on microarray slides. However, said detergents have a beneficial effect on polymerase activity and storage. Hence, leaving the detergent out of the storage buffer or the reaction buffer is also not an ideal solution.

There is a need for alternative means for storing and/or reacting isolated enzymes of bacterial, viral, or archae origin.

The prior art knows PEGylation. This is the act of covalently coupling a polyethylene glycol (PEG) and polyethylene oxide (PEO) structure to another larger molecule, for example, a therapeutic protein (which is then referred to as PEGylated). PEGylated interferon alfa-2a or -2b is a commonly used as injectable treatment for Hepatitis C infection.

PEGylation is a process of attaching the strands of the polymer PEG to molecules, most typically peptides, proteins, and antibody fragments, that can help to meet the challenges of improving the safety and efficiency of many therapeutics. It produces alterations in the physiochemical properties including changes in conformation, electrostatic binding, hydrophobicity etc. These physical and chemical changes increase systemic retention of the therapeutic agent. Also, it can influence the binding affinity of the therapeutic moiety to the cell receptors and can alter the absorption and distribution patterns.

PEGylation, by increasing the molecular weight of a molecule, can impart several significant pharmacological advantages over the unmodified form, such as: improved drug solubility, reduced dosage frequency, without diminished efficacy with potentially reduced toxicity, extended circulating life or increased drug stability. United States Patent 4,179,337 relates to such applications.

However, so far industrially used enzymes, in particular such enzymes which may modify or replicate a nucleic acid have not been PEGylated.

### SUMMARY OF THE INVENTION

The inventors have astonishingly found that certain industrially used enzymes may be modified by coupling a polymer and that enzymes modified in such a way have beneficial features which are not be expected from the prior art. One advantage of such a modification is that enzymes need not be used and/or stored in detergents but retain and/or exhibit activity also in the absence of said detergents.

The invention relates to an isolated, active enzyme of bacterial, fungal, viral, or archae origin, wherein the enzyme is coupled with at least one polymer having a molecular weight between about 500 to about 20,000 daltons selected from the group consisting of polyethylene glycol and polypropylene glycol.

Herein "bacterial, fungal, viral, or archae origin" means that the enzyme may be isolated from said organisms. However, the invention also encompasses such enzymes which are recombinantly produced and/or modified. Modifications include but are not limited to such amino acid changes which enhance the binding of the polymer according to the invention. Modifications include changes of the amino acid sequence which otherwise influence or enhance the activity of the enzyme.

The enzymes according to the invention may be used in industrial methods or *in-vitro* applications such as *in vitro* diagnostics.

### DETAILED DESCRIPTION OF THE INVENTION

The invention relates to an active enzyme of bacterial, fungal, viral, or archae origin, wherein the enzyme is coupled with at least one polymer having a molecular weight between about 500 to about 20,000 daltons selected from the group consisting of polyethylene glycol and polypropylene glycol.

The enzymes according to the invention (see some example enzymes in Table 2) may be used in industrial methods or in *in-vifro* applications a number of which are shown in Table 1.

**Table 1**

| **Application** | **Enzymes used** | **Uses** |
|---|---|---|
| Baking industry | Fungal alpha-amylase enzymes are normally inactivated at about 50 degrees Celsius, but are destroyed during the baking process. | Catalyze breakdown of starch in the flour to sugar. Yeast action on sugar produces carbon dioxide. Used in production of white bread, buns, and rolls. |
| | Proteases | Lowers the protein level of flour. |
| Baby foods | Trypsin | Predigestion of baby food |
| Brewing industry | Enzymes from barley are released during the mashing stage of beer production. | Degrades starch and proteins to produce simple sugars. |
| | Industrially produced barley enzymes | Widely used in the brewing process to substitute for the natural enzymes found in barley. |
| | Amylase, glucanases, proteases | Splits polysaccharides and proteins in the malt. |
| | Betaglucanases and arabinoxylanases | Improve the beer filtration characteristics. |
| | Amyloglucosidase and pullulanases | Used for low-calorie beer and adjustment of fermentability. |
| | Proteases | Used to remove cloudiness produced during storage of beers. |
| | Acetolactatedecarboxylase (ALEC) | Used to avoid the formation of diacetyl |
| Fruit juices | Cellulases, pectinases | Used to clarify fruit juices |
| Dairy industry | Rennin, derived from the stomachs of young ruminant animals (like calves and lambs). | Used in the manufacture of cheese, and to hydrolyze proteins. |
| | Microbially produced enzyme | Use in the dairy industry. |
| | Lipases | Is implemented during the production of Roquefort cheese to enhance the ripening of the blue-mould cheese. |
| | Lactases | Break down lactose to glucose and galactose. |
| Meat tenderizers | Papain | To soften meat for cooking. |
| Starch industry | Amylases, amyloglucosideases and glucoamylases | Converts starch into glucose and various syrups. |
| | Glucose isomerase | Converts glucose into fructose in production of high fructose syrups from starchy materials. |
| Paper industry | Amylases, xylanases, cellulases and ligninases | Degrade starch to lower viscosity, aiding sizing and coating paper, Xylanases reduce bleach required for decolorising; cellulases smooth fibers, enhance water drainage, and promote ink removal; lipases reduce pitch and lignin-degrading enzymes remove lignin to soften paper. |
| Biofuel industry | Cellulases | Used to break down cellulose into sugars that can be fermented (see cellulosic ethanol). |
| | Ligninases | Use of lignin waste |
| Biological detergent | Primarily proteases, produced in proteases, produced in an extracellular form from bacteria | Used for presoak conditions and direct liquid applications helping with removal liquid applications helping with removal of protein stains from clothes. |
| | Amylases | Detergents for machine dish washing to remove resistant starch residues. |
| | Lipases | Used to assist in the removal of fatty and oily stains. |
| | Cellulases | Used in biological fabric conditioners. |
| Contact lens cleaners | Proteases | To remove proteins on contact lens to prevent infections. |
| Rubber industry | Catalase | To generate oxygen from peroxide to convert latex into foam rubber. |
| Photographic industry | Protease (ficin) | Dissolve gelatin off scrap film, allowing recovery of its silver content. |
| Molecular biology | Restriction enzymes, DNA ligase and medicine. polymerases | Used to manipulate DNA in genetic engineering, important in pharmacology, agriculture and Essential for restriction digestion and the polymerase chain reaction. Molecular biology is also important in forensic science. |

| | | |
|---|---|---|
| **Table 1** shows a selection of enzymes used in industry and Molecular biology according to the invention. | | |

Polyethylene glycol (PEG) and polyethylene oxide (PEO) are polymers composed of repeating subunits of identical structure, called monomers, and are the most commercially important polyethers. PEG or PEO refers to an oligomer or polymer of ethylene oxide. The two names are chemically synonymous, but historically PEG has tended to refer to shorter polymers, PEO to longer. Both are prepared by polymerization of ethylene oxide.

The numbers that are often included in the names of PEGs and PEOs indicate their average molecular weights, e.g. a PEG with n = 80 would have an average molecular weight of approximately 3,500 daltons and would be labeled PEG 3,500. Most PEGs and PEOs include molecules with a distribution of molecular weights, *i.e.* they are polydisperse. The size distribution can be characterized statistically by its weight average molecular weight (Mw) and its number average molecular weight (Mn), the ratio of which is called the polydispersity index (Mw/Mn). Mw and Mn can be measured by mass spectroscopy.

In a preferred embodiment the enzyme carries at least a nucleic acid modifying or replicating activity.

Such enzymes are for example, nucleases and ligases. Nucleases cut DNA strands by catalyzing the hydrolysis of the phosphodiester bonds. Nucleases that hydrolyse nucleotides from the ends of DNA strands are called exonucleases, while endonucleases cut within strands. The most frequently-used nucleases are the restriction endonucleases, which cut DNA at specific sequences.

Enzymes called DNA ligases can rejoin cut or broken DNA strands, using the energy from either adenosine triphosphate or nicotinamide adenine dinucleotide.

The enzymes according to the invention include topoisomerases and helicases. Topoisomerases are enzymes with both nuclease and ligase activity. These proteins change the amount of supercoiling in DNA. Some of these enzymes work by cutting the DNA helix and allowing one section to rotate, thereby reducing its level of supercoiling; the enzyme then seals the DNA break. Other types of these enzymes are capable of cutting one DNA helix and then passing a second strand of DNA through this break, before rejoining the helix. Helicases use the chemical energy in nucleoside triphosphates, predominantly ATP, to break hydrogen bonds between bases and unwind the DNA double helix into single strands.

Polymerases are particularly preferred according to the invention. Polymerases are enzymes that synthesise polynucleotide chains from nucleoside triphosphates. They function by adding nucleotides onto the 3' hydroxyl group of the previous nucleotide in the DNA strand. Polymerases are classified according to the type of template that they use.

In DNA replication, a DNA-dependent DNA polymerase makes a DNA copy of a DNA sequence. Accuracy is vital in this process, so many of these polymerases have a proofreading activity. Here, the polymerase recognizes the occasional mistakes in the synthesis reaction by the lack of base pairing between the mismatched nucleotides. If a mismatch is detected, a 3' to 5' exonuclease activity is activated and the incorrect base removed. RNA-dependent DNA polymerases are a specialised class of polymerases that copy the sequence of an RNA strand into DNA. They include reverse transcriptase. Transcription is carried out by a DNA-dependent RNA polymerase that copies the sequence of a DNA strand into RNA.

Thus, in one embodiment of the invention such an activity may be selected from the group comprising enzymes with exonuclease activity, enzymes with endonuclease activity, enzymes with polymerising activity, enzymes with methyltransferase activity, enzymes with recombinase activity, enzymes with polynucleotide kinase activity, enzymes with phosphatase activity and enzymes with sulfurylase activity.

The polymer may have a molecular weight between about 500 and 50,000 daltons.

It is preferred that the polymer has a molecular weight between about 750 and 20,000 daltons, more preferable 50 and 10,000 daltons. When pegylating polymerases it is preferred that the polymer has a molecular weight of between about 2,000 and 8,000 daltons.

It is preferred that the enzyme carries between 1 and 100 polymer moieties per enzyme molecule.

It is preferred that the polymer is polyethylene glycol.

According to the invention it is preferred that the polymer has at least one functional endgroup, or one homobifunctional endgroup or one heterobifunctional endgroup.

It is further preferred that the polymer has at least one heterobifunctional endgroup selected from the group of maleimide, vinyl sulphones, pyridyl disulphide, amine, carboxylic acids and NHS esters. Maleimide is most preferred.

It is preferred that the polymer is bound to a reactive amino acid selected from the group of lysine, cysteine, histidine, arginine, aspartic acid, glutamic acid, serine, threonine and tyrosine.

It is particularly preferred that the polymer is bound to a reactive amino acid located close to the N-terminal end of the enzyme or close to the C-terminal end of the enzyme.

It is preferred that the polymer is bound to the N-terminal amino group and/or the C-terminal carboxylic acid. This is advantageous because often such binding reduces any negative influence on enzyme activity.

In a preferred embodiment the enzyme is selected from the group of fungal alpha-amylase, protease, trypsin, amylase, glucanase, betaglucanase, arabinoxylanases, amyloglucosidase, pullulanases, acetolactatedecarboxylase (ALDC), pectinases, rennin, lipases, lactases, papain, glucoamylases, glucose isomerase, xylanase, cellulase, ligninase, restriction enzymes, DNA ligase, polymerase, a ligase, an endonuclease, an exonuclease, methyltransferase, recombinase, polynucleotide kinase, phosphatases sulfurylases.

In a preferred embodiment the enzyme is an enzyme which has a nucleic acid as a substrate.

In one aspect of the invention the enzyme is an *E*. *coli* Uracil-N-Glycosylase.

In another aspect of the invention the enzyme is a polymerase.

It is most preferred that the polymerase is a DNA-dependent DNA polymerase, an RNA-dependent DNA polymerase or an RNA-dependent RNA polymerase. Table II discloses preferred enzymes as well as enzymes which have been altered according to the invention.

SEQ ID NO. 1 discloses the wildtype Taq DNA sequence, SEQ ID NO. 2 the corresponding amino acid sequence. This protein may be coupled to a polymer according to the invention. Alternatively, the protein may be modified by incorporating amino acids that serve in binding the polymer of the invention. This has been done for

### Thermus eggertssonii (SEQ I D NOs. 5 to 8).

**Table II**

| | |
|---|---|
| SEQ ID NO. 1 | *Thermus aquaticus -* complete nucleotide sequence of the polA gene - (D32013) |
| SEQ ID NO. 2 | *Thermus aquaticus -* complete amino acid sequence of the DNA polymerase I - (D32013) |
| SEQ ID NO. 3 | *Thermus eggertssonii* wild type- complete nucleotide sequence of the polA gene |
| SEQ ID NO. 4 | *Thermus eggertssonii* wild type- complete amino acid sequence of the DNA polymerase I |
| SEQ ID NO. 5 | *Thermus eggertssonii* mutant G834C --complete nucleotide sequence |
| SEQ ID NO. 6 | *Thermus eggertssonii* mutant G834C --complete amino acid sequence |
| SEQ ID NO. 7 | *Thermus eggertssonii* mutant 1825C --complete nucleotide sequence |
| SEQ ID NO. 8 | *Thermus eggertssonii* mutant I825C --complete amino acid sequence |
| SEQ ID NO. 9 | *E. coli* Uracil-N-Glycosylase-complete nucleotide sequence |
| SEQ ID NO. 10 | *E*. *coli* Uracil-N-Glycosylase --complete amino acid sequence |

It is further preferred that the enzyme is from a thermophilic organism. If the enzyme is a polymerase it is particularly preferred that the polymerase is from a thermophilic organism.

It is preferred that the polymerase according to the invention is selected from the group of genera of *Thermus, Aquifex, Thermotoga, Thermocridis, Hydrogenobacter, Thermosynchecoccus* and *Thermoanaerobacter.*

It is preferred that the polymerase according to the invention is selected from the group of organisms of *Aquifex aeolicus, Aquifex pyogenes, Thermus thermophilus, Thermus aquaticus, Thermotoga neapolitana, Thermus pacificus* and *Thermotoga maritima.*

DNA polymerases can be subdivided into seven different families: A, B, C, D, X, Y, and RT. For example, prokaryotic DNA polymerase I is a family A polymerase that mediates the process of DNA repair, while polymerase III is the primary enzyme involved with bacterial DNA replication. Discovered by Arthur Kornberg in 1956,DNA polymerase I was the first known DNA polymerase, and was initially characterized in *E. coli,* although it is ubiquitous in prokaryotes. It is often referred to as simply Pol I. Pol I and its derivatives, such as klenow fragment from E.Coli Pol I and *Thermus aquaticus* Pol I, are widely used in molecular biology research.. In the present invention it is preferred that the enzyme is a pol-A type polymerase.

It is preferred that the enzyme according to the invention is encoded by a nucleic acid according to SEQ ID NO. 1 (Taq) or shares over 80 %, over 85 %, over 90 % over 95°%, or most preferentially over 98 % sequence identity with SEQ ID NO. 1.

It is preferred that the enzyme according to the invention has an amino acid sequence according to SEQ ID NO. 2 (Taq) or shares over 85 %, over 90 % over 95 %, or most preferentially over 98 % sequence identity with SEQ ID NO. 2.

It is preferred that the enzyme according to the invention is encoded by a nucleic acid according to SEQ ID NO. 3 (Teg) or shares over 80 %, over 85 %, over 90 % over 95°%, or most preferentially over 98 % sequence identity with SEQ ID NO. 3.

It is preferred that the enzyme according to the invention has an amino acid sequence according to SEQ ID NO. 4 (Teg) or shares over 85 %, over 90 % over 95 %, or most preferentially over 98 % sequence identity with SEQ ID NO. 4.

It is preferred that the enzyme according to the invention is encoded by a nucleic acid according to SEQ ID NO. 5 (*Thermus eggertssonii* mutant G834C) or shares over 80 %, over 85 %, over 90 % over 95 %, or most preferentially over 98 % sequence identity with SEQ ID NO. 5.

It is preferred that the enzyme according to the invention has an amino acid sequence according to SEQ ID NO. 6 *(Thermus eggertssonii* mutant G834C) or shares over 85 %, over 90 % over 95 %, or most preferentially over 98 % sequence identity with SEQ ID NO. 6.

It is preferred that the enzyme according to the invention is encoded by a nucleic acid according to SEQ ID NO. 7 *(Thermus eggertssonii* mutant 1825C) or shares over 80%, over 85 %, over 90 % over 95 %, or over most preferentially over 98% sequence identity with SEQ ID NO. 7.

It is preferred that the enzyme according to the invention has an amino acid sequence according to SEQ ID NO. 8 *(Thermus eggertssoni* mutant 1825C) or shares over 85 %, over 90 % over 95 %, or most preferentially over 98 % sequence identity with SEQ ID NO. 8.

The enzymes according to the invention which are modified by the addition of the polymer according to the invention may have also other modifications. In one embodiment, the variant Teg DNA polymerase 1 (which carries a polymer) comprises an amino acid sequence having a substitution at position 679 of SEQ ID NO. 4 replacing the glutamic acid residue by a positively charged amino acid such as lysine or arginine. Analysis of the three dimensional structure of Taq DNA polymerase I bound to a DNA substrate has shown that the negative charge of the glutamic acid at the corresponding position (681) in the Taq DNA polymerase sequence (SEQ ID NO.°2) contacts the negatively charged phosphate backbone of the priming strand in the DNA substrate. That contact creates an electrostatic repulsion effect limiting the extension rate and processivity of the polymerase. Mutant variants carrying a lysine instead of glutamic acid at the position have shown faster extension rates and better processivity. Variant Teg DNA polymerases with those features are desirable for various applications, such as fast PCR, DNA sequencing, amplification of long target sequences. The invention also relates to other pol-A type polymerases with said mutation. Upon alignment the person skilled in the art will identify the respective amino acid.

The polymerases according to the invention which are modified by the addition of the polymer according to the invention may have also further modifications. In one embodiment, the variant Teg DNA polymerase I comprises an amino acid sequence having a substitution at position 683 of SEQ ID NO. 4 replacing the glutamic acid residue by a positively charged amino acid such as lysine or arginine. Analysis of the three dimensional structure of Taq DNA polymerase I bound to a DNA substrate has shown that the negative charge of the glutamic acid at the corresponding position (681) in the Taq DNA polymerase sequence (SEQ ID NO. 2) contacts the negatively-charged phosphate backbone of the priming strand in the DNA substrate. That contact creates an electrostatic repulsion effect limiting the extension rate and processivity of the polymerase. Mutant variants carrying a lysine instead of glutamic acid at the position have shown faster extension rates and better processivity. Variant Teg DNA polymerases with those features are desirable for various applications, such as fast PCR, DNA sequencing, amplification of long target sequences.

The polymerases according to the invention which are modified by the addition of the polymer according to the invention may have also further modifications. In one embodiment, a variant Teg DNA polymerase I comprises an amino acid sequence having single or combined substitutions at the positions 612-613 of SEQ ID NO. 4. In one embodiment, a variant Teg DNA polymerase I comprises an amino acid sequence having single or combined substitutions at the positions 616-617 of SEQ ID NO. 4. Random mutagenesis experiments performed on Taq and *E. coli* DNA polymerase I have shown that the amino acid residues at the corresponding positions in their sequence control discrimination between rNTPs and dNTPs as polymerization substrate. They also control discrimination between RNA- or DNA-primed DNA templates, templates with base mismatches at the 3'-terminus of the primer and perfectly annealed primers and between labeled and non-labeled dNTP substrates. Based on the nature of the substitution(s) at these positions, a number of variant Teg DNA Pol I can be provided with useful features for different applications. Variants with increased discrimination against the extension of mismatched primers are useful for allele-specific PCR. Variants with increased affinity for labeled dNTP substrates are useful for fluorescent DNA sequencing and real-time PCR.

The inventors have found that certain enzymes which have been adapted for better pegylation have very good properties. The invention thus also relates to a polymerase encoded by a nucleic acid according to SEQ ID NO. 5 or SEQ ID NO. 7. The invention also relates to a polymerase with an amino acid sequence according to SEQ ID NO. 6 and SEQ ID NO. 8.

In another embodiment of the invention, the variant of Teg DNA Pol I is based on the knowledge that a single residue in DNA polymerases of *Thermus aquaticus* DNA polymerase I family is critical for distinguishing between deoxy- and dideoxyribonucleotides (Taber, S., Richardson, C.C., Proc. Natl. Acad. Sci. USA, 1995, July 3, 92 (14): 6339-43, A single residue and DNA polymerase of the Escherichia coli DNA polymerase I family is critical for distinguishing between deoxy- and dideoxyriboncleotides). In a preferred embodiment, any Pol I variant according to the invention comprises an amino acid sequence having a substitution residue in place of a wildtype phenylalanine in a position corresponding to position 665 of SEQ ID NO. 2. In a preferred embodiment, the substitution residue is a tyrosine. In a preferred embodiment, the Pol I variant comprises an amino acid sequence having a substitution residue in place of a wildtype phenylalanine in a position corresponding to position 669 of Taq. In a preferred embodiment, the substitution residue is a tyrosine.

In one embodiment, the variant Teg DNA Pol I which carries a polymer has four additional amino acid residues Met, Pro, Arg/Lys and Gly at the N-terminus of the amino acid sequence set forth in SEQ ID NO. 4. Based on the deciphered three dimensional structure of Taq DNA polymerase bound to DNA substrate these three additional N-terminal residues are a part of the DNA-binding site in the N-terminal nuclease domain. In the absence of the additional N-terminal amino acids the Teg DNA polymerase has a weakened binding affinity and strength towards its DNA substrate. Teg DNA Pol I variants with strengthened DNA substrate binding properties have better processivity and a faster extension rate than Teg DNA Pol I with the wildtype sequence. Improved processivity and faster extension rates are important functional features of thermostable DNA polymerases used to perform the polymerase chain reaction (PCR) application. They allow for amplification of longer target sequences with higher sensitivity requiring less DNA template in the sample. The additional proline residue in position 2 of the variant Teg DNA Pol I in this embodiment stabilizes the recombinant polymerase against N-terminal degradation by endogenous cytoplasmic proteinases of the *E. coli* host cells according to the rules of stabilizing N-terminal amino acid residues in *E. coli* well established in the prior art.

The enzyme according to the invention may be a fusion protein. Teg DNA Pol I proteins of the invention also include DNA Pol I fusion proteins that comprise a Teg DNA Pol I protein fused to a non-Teg DNA Pol I protein moiety. In one embodiment, a DNA Pol I fusion protein comprises an exonuclease domain of a Teg DNA Pol I protein of the invention. In one embodiment, a DNA Pol I fusion protein comprises a polymerase domain of a Teg DNA Pal I protein of the invention. DNA Pol I fusion proteins of the invention may include moieties that, for example, provide for purification, or contribute to the altered thermostability or altered catalytic activity of a DNA Pol I fusion protein as compared to a Teg DNA Pol I protein.

It is preferred that at least one polymer is coupled to an amino acid that is not present in the amino acid sequence of the enzyme in its natural state but has been incorporated into the enzyme either in addition to an existing amino acid or at the position of an existing amino acid.

It is preferred that at least one polymer is coupled to an amino acid selected from the group of lysine and cysteine.

The amino acid is ideally surface exposed according to the invention.

Polymerase according to the invention, wherein the at least one polymer is coupled to one or more cysteine and/or lysine residues that have been incorporated into the polymerase at a position corresponding to the position of the following amino acids of Taq polymerase Leu⁴⁶¹, Ala⁵²¹, Gly⁶⁴⁸ , Ala⁶⁵³, Ser⁶⁷⁹, Ala⁶⁸³, Ser⁶⁹⁹, Ser⁷³⁹ Ala⁸¹⁴, Ser⁸²⁹ and Glu⁸³². The complete Taq amino acid sequence is shown in SEQ ID NO. 2.

The invention also relates to methods for modifying or amplifying a nucleic acid, wherein the nucleic acid to be modified is present in a reaction mixture comprising an enzyme according to the invention. The methods comprise subjecting a nucleic acid molecule to a reaction mixture comprising an enzyme of the invention. Preferably the method is an amplification reaction and the enzyme is a polymerase.

In a preferred embodiment, the nucleic acid molecule used in the amplification method is DNA. In a preferred embodiment, the DNA molecule is double stranded. In other embodiments, the DNA molecule is single stranded. In a preferred embodiment, the double stranded DNA molecule is a linear DNA molecule. In other embodiments, the DNA molecule is non-linear, for example circular or supercoiled DNA.

In a preferred embodiment, the amplification method is a thermocycling amplification method useful for amplifying a nucleic acid molecule, preferably DNA, which is preferably double stranded, by a temperature-cycled mode. In a preferred embodiment, the method involves subjecting the nucleic acid molecule to a thermocycling amplification reaction in a thermocycling amplification reaction mixture. The thermocycling amplification reaction mixture comprises a Teg DNA Pol I protein of the invention.

In a preferred embodiment, the amplification method is a PCR method. In one embodiment, the method is a degenerate PCR method. In one embodiment, the method is a real-time PCR method.

In one embodiment, the invention provides reaction mixtures for nucleic acid amplification, which comprise a Teg DNA Pol I protein or a polymerase which are modified with the polymer of the invention. Preferred reaction mixtures of the invention are useful for DNA amplification. In a preferred embodiment, the reaction mixture is a thermocycling reaction mixture useful for thermocycling amplification reactions.

Amplification reaction mixtures may include additional reagents, such as, but not limited to, dNTPs, primers, buffer, and/or stabilizers.

In one embodiment, the invention provides reaction mixtures for amplifying nucleic acids using degenerate primers in PCR, which are useful for the amplification of homologous sequence targets containing nucleotide polymorphisms. The reaction mixtures comprise a Teg DNA Pol I protein or a polymerase which is modified with the polymer of the invention. Reaction mixtures for PCR with degenerate primers may include additional reagents such as, but not limited to, dNTPs, degenerate primers, buffer, and/or stabilizers.

In a preferred embodiment, the reaction mixture comprises a Teg DNA Pol I protein of the invention, wherein the Teg DNA Pol I is present in the reaction mixture at a concentration of not less than 120 pg/µL, more preferably not less than 140 pglµL, more preferably not less than 160 pg/µL, more preferably not less than 180 pg/µL, more preferably not less than 200 pg/µL, more preferably not less than 400 pg/µL, more preferably not less than 600 pg/µL.

In a preferred embodiment, the reaction mixture comprises a zwitterionic buffer. In a preferred embodiment, the zwitterionic buffer has a pH between about pH 7.5-8.9. In a preferred embodiment, the buffer comprises a combination of an organic zwitterionic acid and an organic zwitterionic base, potassium ions, and magnesium ions. In an especially preferred embodiment, the reaction mixture comprises 30 mM Bicine, 59 mM Tris, 50 mM KCI, 2 mM magnesium acetate.

In one embodiment, the invention provides reaction mixtures for amplifying nucleic acids, which are useful in PCR reactions with real time product detection. The real-time reaction mixtures comprise a polymerase of the invention preferably a Teg DNA Pol I of the invention. The real-time PCR reaction mixtures may include other reagents, including, but not limited to, dNTPs, fluorescent probes, primers, buffer, stabilizers, nucleic acid-binding dye(s) and/or passive reference dye(s).

In a preferred embodiment, the reaction mixture comprises a Teg DNA Pol I, wherein the thermostable Teg Polymerase I is present in the reaction mixture at a concentration of not less than 120 pg/µL, more preferably not less than 140 pg/µL, more preferably not less than 160 pg/µL, more preferably not less than 180 pg/µL, more preferably not less than 200 pg/µL, more preferably not less than 400 pg/µL, more preferably not less than 600 pg/µL.

In a preferred embodiment, the reaction mixture comprises a zwitterionic buffer. In a preferred embodiment, the zwitterionic buffer has a pH between about pH 7.5-8.9. In a preferred embodiment, the buffer comprises a combination of an organic zwitterionic acid and an organic zwitterionic base, potassium ions, and magnesium ions. In an especially preferred embodiment, the reaction mixture comprises a buffer comprising 40 mM Bicine, 90 mM Tris, 40 mM KCI, 4 mM magnesium acetate, and 100 mM sorbitol.

In another preferred embodiment, the reaction mixture comprises a buffer comprising 25 mM Taps, 0.05 mg/ mL Anti-freeze Protein I, 10.3 mM Tris, 50 mM KCI, 5 mM magnesium acetate, 100 mM sorbitol, and 0.2 mg/ mL BSA.

In one aspect, the invention provides nucleic acid amplification reaction tubes, which comprise a polymerase of the invention preferably a Teg DNA Pol I in a nucleic acid amplification reaction mixture disclosed herein.

In a preferred embodiment, the amplification reaction tubes are thermocycling amplification reaction tubes, which comprise a Teg DNA Pol I in a thermocycling amplification reaction mixture disclosed herein.

In a preferred embodiment, the thermocycling amplification reaction tubes are PCR reaction tubes, which comprise a Teg DNA polymerase I in a PCR reaction mixture disclosed herein.

In a preferred embodiment, the PCR reaction tubes are degenerative PCR reaction tubes, which comprise a Teg DNA Pol I in a degenerative PCR reaction mixture disclosed herein.

In another preferred embodiment, the PCR reaction tubes are real-time PCR reaction tubes, which comprise a Teg DNA Pol I in a real-time PCR reaction mixture disclosed herein.

The invention also relates to a kit comprising an enzyme according to the invention.

In one aspect, the invention provides a nucleic acid amplification kit useful for amplifying nucleic acid, preferably DNA, which is preferably double stranded, which comprises a polymerase of the invention preferably a Teg DNA Pol I disclosed herein. In a preferred embodiment, the amplification kit comprises an amplification reaction mixture disclosed herein.

In a preferred embodiment, the amplification kit is a thermocycling amplification kit useful for amplifying nucleic acids, preferably DNA, which is preferably double stranded, by a temperature-cycled mode. The thermocycling amplification kit comprises a Teg DNA Pol I disclosed herein. Preferably, the thermocycling amplification kit comprises a thermocycling amplification reaction mixture disclosed herein.

In a preferred embodiment, the thermocycling amplification kit is a PCR kit for amplifying nucleic acids, preferably DNA, which is preferably double-stranded, by PCR. The PCR kit comprises a polymerase according to the invention preferably a Teg DNA Pol I disclosed herein. Preferably the PCR kit comprises a PCR reaction mixture disclosed herein.

In a preferred embodiment, the PCR kit is a degenerative PCR kit, preferably comprising a degenerative PCR reaction mixture disclosed herein.

In another preferred embodiment, the PCR kit is a real-time PCR kit, preferably comprising a real-time PCR reaction mixture disclosed herein.

In a preferred embodiment, a nucleic acid amplification kit provided herein comprises a nucleic acid amplification reaction mixture, which comprises an amount of a polymerase according to the invention preferably Teg DNA Pol I such that the reaction mixture can be combined with template DNA, primer(s) and/or probe(s) hybridizable thereto, and optionally appropriately diluted to produce a charged reaction mixture, wherein the thermostable DNA Pol I is capable of amplifying the DNA template by extending the hybridized primer(s).

### FIGURE CAPTIONS

### FIGURE 1

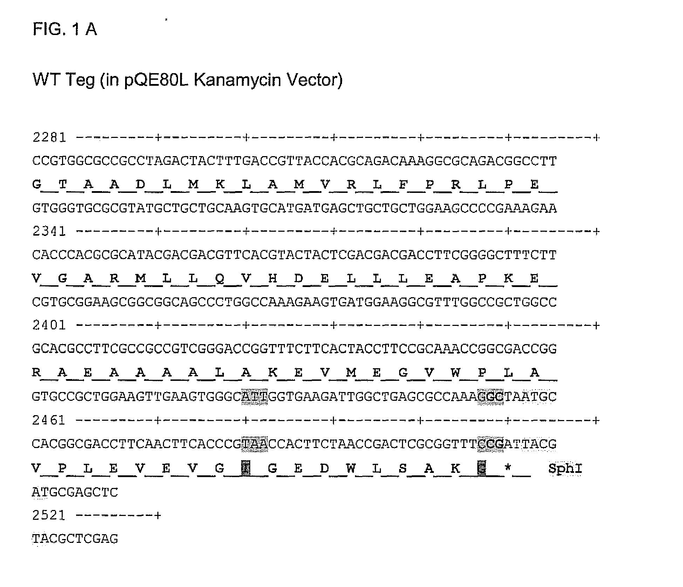

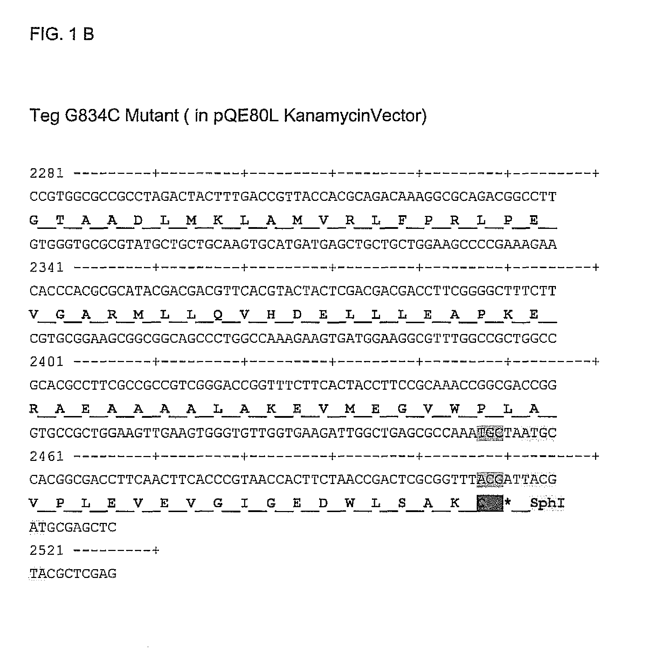

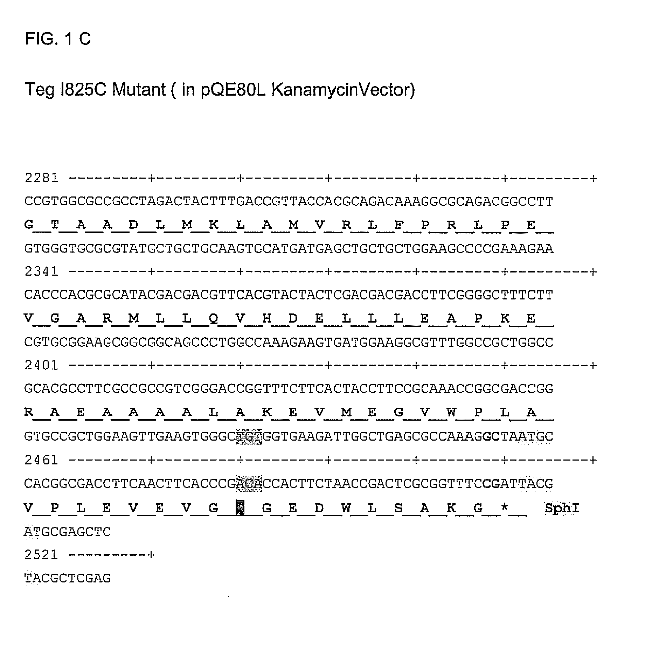

Figure 1A shows WT Teg (in pQE80L kanamycin vector). Figure 1B shows Teg G834C mutant (in pQE80L kanamycin vector). Figure 1C shows Teg 1825C mutant (in pQE80L kanamycin vector).

### FIGURE 2

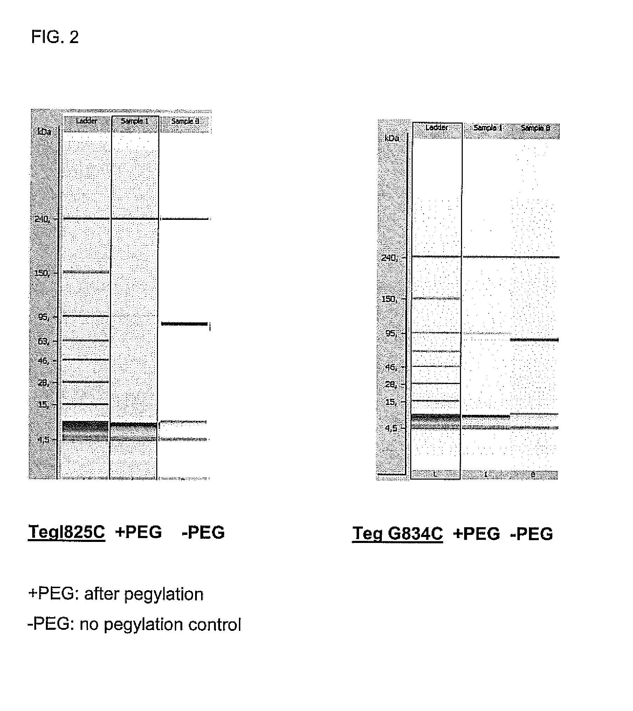

Figure 2 shows gel images which show that Teg mutants were successfully conjugated with methoxypolyethylene glycol 5,000 maleimide.

### FIGURE 3

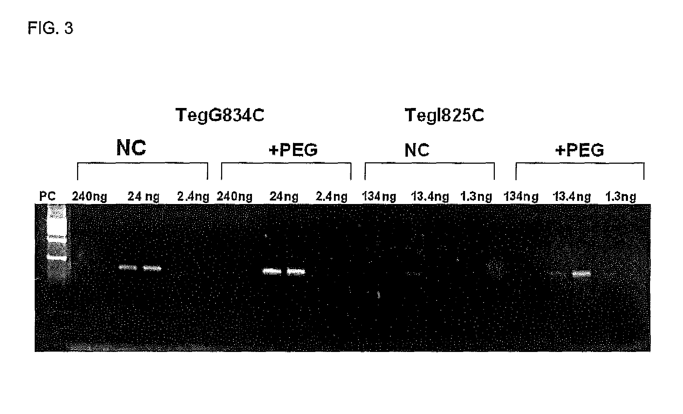

Figure 3 shows that pegylated Teg DNA polymerase produced more PCR product in the absence of detergents.

### EXAMPLES

### Pegylation of Thermus eggertssonii (Teg) Polymerase Enhances its Performance in PCR

Teg, a thermostable DNA polymerase, does not have any cysteine amino acid in its wildtype form. Therefore, it is possible to carry out site-specific pegylation with Teg mutants containing cysteine and PEG-maleimide. We used site-directed mutagenesis method (QuikChange XL Site-Directed Mutagenesis Kit, Stratagene) to introduce cysteine on the C-terminus of the protein: 1. Teg 1825C, where the 825th amino acid of Teg, isoleucine, was changed to cysteine; 2. Teg G834C, where the last amino acid glycine of Teg was changed to cysteine. (Figure 1, wildtype and mutant sequences of the C-terminus of Teg.)

The two Teg mutants, Teg I825C and Teg G834C, were expressed in *E. coli* cells, purified, and resuspended in 100 µM HEPES buffer at the concentrations of 6.7 µg/µl and 12 µg/µl, respectively. 10 µl each of Teg I825C and Teg G834C was mixed with 2.4 µl and 4 µl, respectively, of the 10 mg/ml PEG-maleimide (Methoxypolyethylene glycol 5,000 maleimide, 63187, Fluka/Sigma) to ensure the same molecular ratio of polymerase: PEG for both mutants. The Teg/PEG mixture was diluted with 100 µM HEPES to a final volume of 500 µl and incubated at 4°C overnight. Successful pegylation (>90 % of the protein was pegylated) was demonstrated by the appearance of an additional protein band with lower electrophoresis mobility in the Agilent protein gel (Figure 2).

Next, we examined the stability of the pegylated Teg mutants in a PCR reaction. Detergents are normally required to stabilize thermostable polymerase in the PCR. 1°µl from the not diluted, 1:10, or 1:100 diluted pegylation reaction (the amount of the Teg I825C and Teg G834C was indicated in Figure 3) was used in 25 µl PCR to amplify a 750-bp amplicon from the human genomic DNA in the absence of detergents.

Negative controls (NC) are unpegylated Teg proteins with PEG-maleimide directly added to the PCR reaction. PCR was performed in duplicates. As shown in Figure 3, unpegylated Teg generated no or very low level of PCR product in the absence of detergents. However, pegylated Teg mutants produced higher yield of PCR products compared to negative controls.

Organization Applicant
--------------------
Street :
City :
State :
Country : Germany
PostalCode :
PhoneNumber :
FaxNumber :
EmailAddress :
<110> OrganizationName : QIAGEN GMBH

Application Project
-------------------
<120> Title : MODIFIED ENZYMES AND THEIR USES
<130> AppFileReference : N3003 EP BLN
<140> CurrentAppNumber :
<141> CurrentFilingDate : ____-__-__

Sequence
--------
<213> OrganismName : Thermus aquaticus
<400> PreSequenceString :
atgaggggga tgctgcccct ctttgagccc aagggccggg tcctcctggt ggacggccac 60
cacctggcct accgcacctt ccacgccctg aagggcctca ccaccagccg gggggagccg 120
gtgcaggcgg tctacggctt cgccaagagc ctcctcaagg ccctcaagga ggacggggac 180
gcggtgatcg tggtctttga cgccaaggcc ccctccttcc gccacgaggc ctacgggggg 240
tacaaggcgg gccgggcccc cacgccggag gactttcccc ggcaactcgc cctcatcaag 300
gagctggtgg acctcctggg gctggcgcgc ctcgaggtcc cgggctacga ggcggacgac 360
gtcctggcca gcctggccaa gaaggcggaa aaggagggct acgaggtccg catcctcacc 420
gccgacaaag acctttacca gctcctttcc gaccgcatcc acgccctcca ccccgagggg 480
tacctcatca ccccggcctg gctttgggaa aagtacggcc tgaggcccga ccagtgggcc 540
gactaccggg ccctgaccgg ggacgagtcc gacaaccttc ccggggtcaa gggcatcggg 600
gagaagacgg cgaggaagct tctggaggag tgggggagcc tggaagccct cctcaagaac 660
ctggaccggc tgaagcccgc catccgggag aagatcctgg cccacatgga cgatctgaag 720
ctctcctggg acctggccaa ggtgcgcacc gacctgcccc tggaggtgga cttcgccaaa 780
aggcgggagc ccgaccggga gaggcttagg gcctttctgg agaggcttga gtttggcagc 840
ctcctccacg agttcggcct tctggaaagc cccaaggccc tggaggaggc cccctggccc 900
ccgccggaag gggccttcgt gggctttgtg ctttcccgca aggagcccat gtgggccgat 960
cttctggccc tggccgccgc caggggggcc cgggtccacc gggcccccga gccttataaa 1020
gccctcaggg acctgaagga ggcgcggggg cttctcgcca aagacctgag cgttctggcc 1080
ctgagggaag gccttggcct cccgcccggc gacgacccca tgctcctcgc ctacctcctg 1140
gacccttcca acaccacccc cgaggggtg gcccggcgct acggcgggga gtggacggag 1200
gaggcggggg agcgggccgc cctttccgag aggctcttcg ccaacctgtg gggaggctt 1260
gagggggagg agaggctcct ttggctttac cgggaggtgg agaggcccct ttccgctgtc 1320
ctggcccaca tggaggccac gggggtgcgc ctggacgtgg cctatctcag ggccttgtcc 1380
ctggaggtgg ccgaggagat cgcccgcctc gaggccgagg tcttccgcct ggccggccac 1440
cccttcaacc tcaactcccg ggaccagctg gaaagggtcc tctttgacga gctagggctt 1500
cccgccatcg gcaagacgga gaagaccggc aagcgctcca ccagcgccgc cgtcctggag 1560
gccctccgcg aggcccaccc catcgtggag aagatcctgc agtaccggga gctcaccaag 1620
ctgaagagca cctacattga ccccttgccg gacctcatcc accccaggac gggccgcctc 1680
cacacccgct tcaaccagac ggccacggcc acgggcaggc taagtagctc cgatcccaac 1740
ctccagaaca tccccgtccg caccccgctt gggcagagga tccgccgggc cttcatcgcc 1800
gaggaggggt ggctattggt ggccctggac tatagccaga tagagctcag ggtgctggcc 1860
cacctctccg gcgacgagaa cctgatccgg gtcttccagg aggggcggga catccacacg 1920
gagaccgcca gctggatgtt cggcgtcccc cgggaggccg tggacccct gatgcgccgg 1980
gcggccaaga ccatcaactt cggggtcctc tacggcatgt cggcccaccg cctctcccag 2040
gagctagcca tcccttacga ggaggcccag gccttcattg agcgctactt tcagagcttc 2100
cccaaggtgc gggcctggat tgagaagacc ctggaggagg gcaggaggcg ggggtacgtg 2160
gagaccctct tcggccgccg ccgctacgtg ccagacctag aggcccgggt gaagagcgtg 2220
cgggaggcgg ccgagcgcat ggccttcaac atgcccgtcc agggcaccgc cgccgacctc 2280
atgaagctgg ctatggtgaa gctcttcccc aggctggagg aaatgggggc caggatgctc 2340
cttcaggtcc acgacgagct ggtcctcgag gccccaaaag agagggcgga ggccgtggcc 2400
cggctggcca aggaggtcat ggagggggtg tatcccctgg ccgtgcccct ggaggtggag 2460
gtggggatag gggaggactg gctctccgcc aaggagtga 2499
<212> Type : DNA
<211> Length : 2499
SequenceName : SEQ ID NO. 1 Thermus aquaticus - complete nucleotide

sequence of the polA gene - (D32013)
SequenceDescription :

Sequence
--------
<213> OrganismName : Thermus aquaticus
<400> PreSequenceString :
MRGMLPLFEP KGRVLLVDGH HLAYRTFHAL KGLTTSRGEP VQAVYGFAKS LLKALKEDGD 60
AVIVVFDAKA PSFRHEAYGG YKAGRAPTPE DFPRQLALIK ELVDLLGLAR LEVPGYEADD 120
VLASLAKKAE KEGYEVRILT ADKDLYQLLS DRIHALHPEG YLITPAWLWE KYGLRPDQWA 180
DYRALTGDES DNLPGVKGIG EKTARKLLEE WGSLEALLKN LDRLKPAIRE KILAHMDDLK 240
LSWDLAKVRT DLPLEVDFAK RREPDRERLR AFLERLEFGS LLHEFGLLES PKALEEAPWP 300
PPEGAFVGFV LSRKEPMWAD LLALAAARGG RVHRAPEPYK ALRDLKEARG LLAKDLSVLA 360
LREGLGLPPG DDPMLLAYLL DPSNTTPEGV ARRYGGEWTE EAGERAALSE RLFANLWGRL 420
EGEERLLWLY REVERPLSAV LAHMEATGVR LDVAYLRALS LEVAEEIARL EAEVFRLAGH 480
PFNLNSRDQL ERVLFDELGL PAIGKTEKTG KRSTSAAVLE ALREAHPIVE KILQYRELTK 540
LKSTYIDPLP DLIHPRTGRL HTRFNQTATA TGRLSSSDPN LQNIPVRTPL GQRIRRAFIA 600
EEGWLLVALD YSQIELRVLA HLSGDENLIR VFQEGRDIHT ETASWMFGVP REAVDPLMRR 660
AAKTINFGVL YGMSAHRLSQ ELAIPYEEAQ AFIERYFQSF PKVRAWIEKT LEEGRRRGYV 720
ETLFGRRRYV PDLEARVKSV REAAERMAFN MPVQGTAADL MKLAMVKLFP RLEEMGARML 780
LQVHDELVLE APKERAEAVA RLAKEVMEGV YPLAVPLEVE VGIGEDWLSA KE 832

<212> Type : PRT
<211> Length : 832
SequenceName : SEQ ID NO. 2 Thermus aquaticus - complete amino acid
sequence of the DNA polymerase I - (D32013)
SequenceDescription :

Sequence
--------
<213> OrganismName : Thermus aquaticus
<400> PreSequenceString :
atgggccgtggtatgctgccgctgtttgaaccgaaaggccgtgtgctgctggttgatggccaccatctggcctatcgtaa
ctttttttgcgctgaaaggcctgaccacgagccgtggtgaaccggtgcagggcgtgtatggctttgcgaaaagcctgctga
aagcgctgaaagaggatggcgacgttgttattgtggtgtttgatgcgaaagcgccgtttttttcgtcatgaagcgtacgaa
gcgtataaagcgggccgtgcgccgaccccggaagattttccgcgtcagctggccctgattaaagagctggttgatctgct
gggcctggaacgtctggaagtgccgggctttgaagcggatgatgtgctggccaccctggccaaacaggcggaacgtgaag
gctatgaagtgcgtattctgaccgcggatcgtgacctgtttcagctgctgagcgatcgtattgcggtgctgcatccggaa
ggccatctgattacgccgggctggctgtgggaacgttatggcctgaaaccggaacagtgggtggattttcgtgcgctggc
cggcgatccgagcgataacattccgggcgtgaaaggcattggcgaaaaaaccgcgctgaaactgctgaaagaatggggca
gcctggaaaatctgctgaaaaacctggatcatgtgaaaccgccgagcgtgcgtgaaaaaattctggcccatctggatgat
ctgcgtctgtctcaggagctgtctcgcgttcgtaccgatctgccgctgaaagtggattttaaaaaacgtcgtgaaccgga
tcgtgaaggcctgaaagcgtttctggaacgcctggaatttggcagcctgctgcatgaatttggcctgctggaaagcccgc
tgccggcggaagaggcgccgtggccgccaccggaaggtgcgtttctgggctatcgtctgagccgtccggaaccgatgtgg
gcggagctgctggccctggccgcgagcgcgaaaggtcgtgtgtatcgtgcggaagaaccgtatggcgcgctgcgtggcct
gaaagaagtgcgcggcctgctggctaaagacctggccgtgctggccctgcgtgaaggtctggatctgccgccgaccgatg
atccgatgctgctggcctatctgctggacccgagcaacaccaccccggaaggtgtggcgcgtcgttatggcggcgaatgg
accgaagaagcgggcgaacgcgcggttctgagcgaacgtctgtatgaaaacctgctgggccgtctgcgtggcgaagaaaa
actgctgtggctgtatgaagaagtggaaaaaccgctgagccgtgtgctggcccatatggaagcgaccggcgtgcgtctgg
atgtggcgtatctgaaagccctgagcctggaagtggcggaagaaatgcgtcgtctggaagaagaagtgtttcgtctggcc
ggccatccgtttaacctgaacagccgtgatcagctggaacgtgtgctgtttgatgagctgggcctgccgccgattggcaa
aaccgaaaaaaccggcaaacgtagcaccagcgcggcggttctggaagcgctgcgtgaagcgcatccgattgtggaaaaaa
tcctgcaatatcgtgagctggccaaactgaaaggcacctatattgatccgctgccggccctggtgcatccgaaaaccggc
cgtctgcatacccgttttaaccagaccgcgaccgcgaccggtcgtctgagcagcagcgatccgaacctgcaaaacattcc
ggtgcgtaccccgctgggccagcgtattcgtcgtgcgtttgtggccgaagaaggctatctgctggttgcgctggattaag
ccagattgagctgcgtgttctggcccacctgagcggcgatgaaaatctgattcaggtgtttcaggaaggccgcgatattc
atacccagaccgcgagctggatgtttggcctgccggccgaagcgatcgatccgctgatgcgtcgtgcggcgaaaaccatt
aactttggcgtgctgtatggcatgagcgcgcatcgcctgagccaggagctgagcattccgtacgaagaagcggtggcgtt
tattgatcgttatttccagagctacccgaaagtgaaagcgtggattgaacgtaccctggaagaaggccgtcagcgcggct
atgtggaaaccctgtttggccgtcgtcgttatgtgccggatctgaacgcgcgtgtgaaaagcgttcgtgaagcggcggaa
cgtatggcgtttaacatgccggttcagggcaccgcggcggatctgatgaaactggcaatggtgcgtctgtttccgcgtct
gccggaagtgggtgcgcgtatgctgctgcaagtgcatgatgagctgctgctggaagccccgaaagaacgtgcggaagcgg
cggcagccctggccaaagaagtgatggaggcgtttggccgctggccgtgccgctggaagttgaagtgggcattggtgaag
attggctgagcgccaaaggctaa
<212> Type : DNA
<211> Length : 2503
SequenceName : SEQ ID NO. 3 Thermus eggertssoni wild type- complete
nucleotide sequence of the polA gene

SequenceDescription :

Sequence
--------
<213> OrganismName : Thermus aquaticus
<400> PreSequenceString :
MGRGMLPLFEPKGRVLLVDGHHLAYRNFFALKGLTTSRGEPVQGVYGFAKSLLKAL

KEDGDVVIVVFDAKAPFFRHEAYEAYKAGRAPTPEDFPRQLALIKELVDLLGLERLEVPG

FEADDVLATLAKQAEREGYEVRILTADRDLFQLLSDRIAVLHPEGHLITPGWLWERYGLK

PEQWVDFRALAGDPSDNIPGVKGIGEKTALKLLKEWGSLENLLKNLDHVKPPSVREKILA

HLDDLRLSQELSRVRTDLPLKVDFKKRREPDREGLKAFLERLEFGSLLHEFGLLESPLPA

EEAPWPPPEGAFLGYRLSRPEPMWAELLALAASAKGRVYRAEEPYGALRGLKEVRGLLAK

DLAVLALREGLDLPPTDDPMLLAYLLDPSNTTPEGVARRYGGEWTEEAGERAVLSERLYE

NLLGRLRGEEKLLWLYEEVEKPLSRVLAHMEATGVRLDVAYLKALSLEVAEEMRRLEEEV

FRLAGHPFNLNSRDQLERVLFDELGLPPIGKTEKTGKRSTSAAVLEALREAHPIVEKILQ

YRELAKLKGTYIDPLPALVHPKTGRLHTRFNQTATATGRLSSSDPNLQNIPVRTPLGQRI

RRAFVAEEGYLLVALDYSQIELRVLAHLSGDENLIQVFQEGRDIHTQTASWMFGLPAEAI

DPLMRRAAKTINFGVLYGMSAHRLSQELSIPYEEAVAFIDRYFQSYPKVKAWIERTLEEG

RQRGYVETLFGRRRYVPDLNARVKSVREAAERMAFNMPVQGTAADLMKLAMVRLFPRLPE

VGARMLLQVHDELLLEAPKERAEAAAALAKEVMEGVWPLAVPLEVEVGIGEDWLSAKG

<212> Type : PRT
<211> Length : 834
      SequenceName : SEQ ID NO. 4 Thermus eggertssonii wild type- complete amino
acid sequence of the DNA polymerase I
      SequenceDescription :

Sequence
--------
<213> OrganismName : Thermus aquaticus
<400> PreSequenceString :
atgggccgtg gtatgctgcc gctgtttgaa ccgaaaggcc gtgtgctgct ggttgatggc      60
caccatctgg cctatcgtaa cttttttgcg ctgaaaggcc tgaccacgag ccgtggtgaa      120
ccggtgcagg gcgtgtatgg ctttgcgaaa agcctgctga aagcgctgaa agaggatggc      180
gacgttgtta ttgtggtgtt tgatgcgaaa gcgccgtttt ttcgtcatga agcgtacgaa      240
gcgtataaag cgggccgtgc gccgaccccg gaagattttc cgcgtcagct ggccctgatt      300
aaagagctgg ttgatctgct gggcctggaa cgtctggaag tgccgggctt tgaagcggat      360
gatgtgctgg ccaccctggc caaacaggcg gaacgtgaag gctatgaagt gcgtattctg      420
accgcggatc gtgacctgtt tcagctgctg agcgatcgta ttgcggtgct gcatccggaa      480
ggccatctga ttacgccggg ctggctgtgg gaacgttatg gcctgaaacc ggaacagtgg      540
gtggattttc gtgcgctggc cggcgatccg agcgataaca ttccgggcgt gaaaggcatt      600
ggcgaaaaaa ccgcgctgaa actgctgaaa gaatggggca gcctggaaaa tctgctgaaa      660
aacctggatc atgtgaaacc gccgagcgtg cgtgaaaaaa ttctggccca tctggatgat      720
ctgcgtctgt ctcaggagct gtctcgcgtt cgtaccgatc tgccgctgaa agtggatttt      780
aaaaaacgtc gtgaaccgga tcgtgaaggc ctgaaagcgt ttctggaacg cctggaattt      840
ggcagcctgc tgcatgaatt tggcctgctg gaaagcccgc tgccggcgga agaggcgccg      900
tggccgccac cggaaggtgc gtttctgggc tatcgtctga gccgtccgga accgatgtgg      960
gcggagctgc tggccctggc cgcgagcgcg aaaggtcgtg tgtatcgtgc ggaagaaccg      1020
tatggcgcgc tgcgtggcct gaaagaagtg cgcggcctgc tggctaaaga cctggccgtg      1080
ctggccctgc gtgaaggtct ggatctgccg ccgaccgatg atccgatgct gctggcctat      1140
ctgctggacc cgagcaacac caccccggaa ggtgtggcgc gtcgttatgg cggcgaatgg      1200
accgaagaag cgggcgaacg cgcggttctg agcgaacgtc tgtatgaaaa cctgctgggc      1260
cgtctgcgtg gcgaagaaaa actgctgtgg ctgtatgaag aagtggaaaa accgctgagc      1320
cgtgtgctgg cccatatgga agcgaccggc gtgcgtctgg atgtggcgta tctgaaagcc      1380

ctgagcctgg aagtggcgga agaaatgcgt cgtctggaag aagaagtgtt tcgtctggcc 1440
ggccatccgt ttaacctgaa cagccgtgat cagctggaac gtgtgctgtt tgatgagctg 1500
ggcctgccgc cgattggcaa aaccgaaaaa accggcaaac gtagcaccag cgcggcggtt 1560
ctggaagcgc tgcgtgaagc gcatccgatt gtggaaaaaa tcctgcaata tcgtgagctg 1620
gccaaactga aaggcaccta tattgatccg ctgccggccc tggtgcatcc gaaaaccggc 1680
cgtctgcata cccgttttaa ccagaccgcg accgcgaccg gtcgtctgag cagcagcgat 1740
ccgaacctgc aaaacattcc ggtgcgtacc ccgctgggcc agcgtattcg tcgtgcgttt 1800
gtggccgaag aaggctatct gctggttgcg ctggattaag ccagattgag ctgcgtgttc 1860
tggcccacct gagcggcgat gaaaatctga ttcaggtgtt tcaggaaggc cgcgatattc 1920
atacccagac cgcgagctgg atgtttggcc tgccggccga agcgatcgat ccgctgatgc 1980
gtcgtgcggc gaaaaccatt aactttggcg tgctgtatgg catgagcgcg catcgcctga 2040
gccaggagct gagcattccg tacgaagaag cggtggcgtt tattgatcgt tatttccaga 2100
gctacccgaa agtgaaagcg tggattgaac gtaccctgga agaaggccgt cagcgcggct 2160
atgtggaaac cctgtttggc cgtcgtcgtt atgtgccgga tctgaacgcg cgtgtgaaaa 2220
gcgttcgtga agcggcggaa cgtatggcgt ttaacatgcc ggttcagggc accgcggcgg 2280
atctgatgaa actggcaatg gtgcgtctgt ttccgcgtct gccggaagtg ggtgcgcgta 2340
tgctgctgca agtgcatgat gagctgctgc tggaagcccc gaaagaacgt gcggaagcgg 2400
cggcagccct ggccaaagaa gtgatggagg cgtttggccg ctggccgtgc cgctggaagt 2460
tgaagtgggc attggtgaag attggctgag cgccaaatgc taa 2503
<212> Type : DNA
<211> Length : 2503
SequenceName : SEQ ID NO. 5 Thermus eggertssoni mutant G834C --complete nucleotide sequence
SequenceDescription :

Sequence
--------
<213> OrganismName : Thermus aquaticus
<400> PreSequenceString :
MGRGMLPLFE PKGRVLLVDG HHLAYRNFFA LKGLTTSRGE PVQGVYGFAK SLLKALKEDG 60
DVVIVVFDAK APFFRHEAYE AYKAGRAPTP EDFPRQLALI KELVDLLGLE RLEVPGFEAD 120
DVLATLAKQA EREGYEVRIL TADRDLFQLL SDRIAVLHPE GHLITPGWLW ERYGLKPEQW 180
VDFRALAGDP SDNIPGVKGI GEKTALKLLK EWGSLENLLK NLDHVKPPSV REKILAHLDD 240
LRLSQELSRV RTDLPLKVDF KKRREPDREG LKAFLERLEF GSLLHEFGLL ESPLPAEEAP 300
WPPPEGAFLG YRLSRPEPMW AELLALAASA KGRVYRAEEP YGALRGLKEV RGLLAKDLAV 360
LALREGLDLP PTDDPMLLAY LLDPSNTTPE GVARRYGGEW TEEAGERAVL SERLYENLLG 420
RLRGEEKLLW LYEEVEKPLS RVLAHMEATG VRLDVAYLKA LSLEVAEEMR RLEEEVFRLA 480
GHPFNLNSRD QLERVLFDEL GLPPIGKTEK TGKRSTSAAV LEALREAHPI VEKILQYREL 540
AKLKGTYIDP LPALVHPKTG RLHTRFNQTA TATGRLSSSD PNLQNIPVRT PLGQRIRRAF 600
VAEEGYLLVA LDYSQIELRV LAHLSGDENL IQVFQEGRDI HTQTASWMFG LPAEAIDPLM 660
RRAAKTINFG VLYGMSAHRL SQELSIPYEE AVAFIDRYFQ SYPKVKAWIE RTLEEGRQRG 720
YVETLFGRRR YVPDLNARVK SVREAAERMA FNMPVQGTAA DLMKLAMVRL FPRLPEVGAR 780
MLLQVHDELL LEAPKERAEA AAALAKEVME GVWPLAVPLE VEVGIGEDWL SAKC 834

<212> Type : PRT
<211> Length : 834
SequenceName : SEQ ID NO. 6 Thermus eggertssoni mutant G834C - complete amino acid sequence
SequenceDescription :

Sequence
--------
<213> OrganismName : Thermus aquaticus
<400> PreSequenceString :
atgggccgtg gtatgctgcc gctgtttgaa ccgaaaggcc gtgtgctgct ggttgatggc 60
caccatctgg cctatcgtaa ctttttttgcg ctgaaaggcc tgaccacgag ccgtggtgaa 120
ccggtgcagg gcgtgtatgg ctttgcgaaa agcctgctga aagcgctgaa agaggatggc 180
gacgttgtta ttgtggtgtt tgatgcgaaa gcgccgtttt ttcgtcatga agcgtacgaa 240
gcgtataaag cgggccgtgc gccgaccccg gaagattttc cgcgtcagct ggccctgatt 300
aaagagctgg ttgatctgct gggcctggaa cgtctggaag tgccgggctt tgaagcggat 360
gatgtgctgg ccaccctggc caaacaggcg gaacgtgaag gctatgaagt gcgtattctg 420
accgcggatc gtgacctgtt tcagctgctg agcgatcgta ttgcggtgct gcatccggaa 480
ggccatctga ttacgccggg ctggctgtgg gaacgttatg gcctgaaacc ggaacagtgg 540
gtggattttc gtgcgctggc cggcgatccg agcgataaca ttccgggcgt gaaaggcatt 600
ggcgaaaaaa ccgcgctgaa actgctgaaa gaatggggca gcctggaaaa tctgctgaaa 660
aacctggatc atgtgaaacc gccgagcgtg cgtgaaaaaa ttctggccca tctggatgat 720
ctgcgtctgt ctcaggagct gtctcgcgtt cgtaccgatc tgccgctgaa agtggatttt 780
aaaaaacgtc gtgaaccgga tcgtgaaggc ctgaaagcgt ttctggaacg cctggaattt 840

ggcagcctgc tgcatgaatt tggcctgctg gaaagcccgc tgccggcgga agaggcgccg 900
tggccgccac cggaaggtgc gtttctgggc tatcgtctga gccgtccgga accgatgtgg 960
gcggagctgc tggccctggc cgcgagcgcg aaaggtcgtg tgtatcgtgc ggaagaaccg 1020
tatggcgcgc tgcgtggcct gaaagaagtg cgcggcctgc tggctaaaga cctggccgtg 1080
ctggccctgc gtgaaggtct ggatctgccg ccgaccgatg atccgatgct gctggcctat 1140
ctgctggacc cgagcaacac caccccggaa ggtgtggcgc gtcgttatgg cggcgaatgg 1200
accgaagaag cgggcgaacg cgcggttctg agcgaacgtc tgtatgaaaa cctgctgggc 1260
cgtctgcgtg gcgaagaaaa actgctgtgg ctgtatgaag aagtggaaaa accgctgagc 1320
cgtgtgctgg cccatatgga agcgaccggc gtgcgtctgg atgtggcgta tctgaaagcc 1380
ctgagcctgg aagtggcgga agaaatgcgt cgtctggaag aagaagtgtt tcgtctggcc 1440
ggccatccgt ttaacctgaa cagccgtgat cagctggaac gtgtgctgtt tgatgagctg 1500
ggcctgccgc cgattggcaa aaccgaaaaa accggcaaac gtagcaccag cgcggcggtt 1560
ctggaagcgc tgcgtgaagc gcatccgatt gtggaaaaaa tcctgcaata tcgtgagctg 1620
gccaaactga aaggcaccta tattgatccg ctgccggccc tggtgcatcc gaaaaccggc 1680
cgtctgcata cccgttttaa ccagaccgcg accgcgaccg gtcgtctgag cagcagcgat 1740
ccgaacctgc aaaacattcc ggtgcgtacc ccgctgggcc agcgtattcg tcgtgcgttt 1800
gtggccgaag aaggctatct gctggttgcg ctggattaag ccagattgag ctgcgtgttc 1860
tggcccacct gagcggcgat gaaaatctga ttcaggtgtt tcaggaaggc cgcgatattc 1920
atacccagac cgcgagctgg atgtttggcc tgccggccga agcgatcgat ccgctgatgc 1980
gtcgtgcggc gaaaaccatt aactttggcg tgctgtatgg catgagcgcg catcgcctga 2040
gccaggagct gagcattccg tacgaagaag cggtggcgtt tattgatcgt tatttccaga 2100
gctacccgaa agtgaaagcg tggattgaac gtaccctgga agaaggccgt cagcgcggct 2160
atgtggaaac cctgtttggc cgtcgtcgtt atgtgccgga tctgaacgcg cgtgtgaaaa 2220
gcgttcgtga agcggcggaa cgtatggcgt ttaacatgcc ggttcagggc accgcggcgg 2280
atctgatgaa actggcaatg gtgcgtctgt ttccgcgtct gccggaagtg ggtgcgcgta 2340
tgctgctgca agtgcatgat gagctgctgc tggaagcccc gaaagaacgt gcggaagcgg 2400
cggcagccct ggccaaagaa gtgatggagg cgtttggccg ctggccgtgc cgctggaagt 2460
tgaagtgggc tgtggtgaag attggctgag cgccaaaggc taa 2503
<212> Type : DNA
<211> Length : 2503
SequenceName : SEQ ID NO. 7 Thermus eggertssoni mutant I825C --complete
nucleotide sequence
SequenceDescription :

Sequence
--------
<213> OrganismName : Thermus aquaticus
<400> PreSequenceString :
MGRGMLPLFE PKGRVLLVDG HHLAYRNFFA LKGLTTSRGE PVQGVYGFAK SLLKALKEDG 60
DVVIVVFDAK APFFRHEAYE AYKAGRAPTP EDFPRQLALI KELVDLLGLE RLEVPGFEAD 120
DVLATLAKQA EREGYEVRIL TADRDLFQLL SDRIAVLHPE GHLITPGWLW ERYGLKPEQW 180
VDFRALAGDP SDNIPGVKGI GEKTALKLLK EWGSLENLLK NLDHVKPPSV REKILAHLDD 240
LRLSQELSRV RTDLPLKVDF KKRREPDREG LKAFLERLEF GSLLHEFGLL ESPLPAEEAP 300
WPPPEGAFLG YRLSRPEPMW AELLALAASA KGRVYRAEEP YGALRGLKEV RGLLAKDLAV 360
LALREGLDLP PTDDPMLLAY LLDPSNTTPE GVARRYGGEW TEEAGERAVL SERLYENLLG 420
RLRGEEKLLW LYEEVEKPLS RVLAHMEATG VRLDVAYLKA LSLEVAEEMR RLEEEVFRLA 480
GHPFNLNSRD QLERVLFDEL GLPPIGKTEK TGKRSTSAAV LEALREAHPI VEKILQYREL 540
AKLKGTYIDP LPALVHPKTG RLHTRFNQTA TATGRLSSSD PNLQNIPVRT PLGQRIRRAF 600
VAEEGYLLVA LDYSQIELRV LAHLSGDENL IQVFQEGRDI HTQTASWMFG LPAEAIDPLM 660
RRAAKTINFG VLYGMSAHRL SQELSIPYEE AVAFIDRYFQ SYPKVKAWIE RTLEEGRQRG 720
YVETLFGRRR YVPDLNARVK SVREAAERMA FNMPVQGTAA DLMKLAMVRL FPRLPEVGAR 780
MLLQVHDELL LEAPKERAEA AAALAKEVME GVWPLAVPLE VEVGCGEDWL SAKG 834

<212> Type : PRT
<211> Length : 834
SequenceName : SEQ ID NO. 8 Thermus eggertssoni mutant I825C --complete
amino acid sequence
SequenceDescription :

Sequence
--------
<213> OrganismName : Thermus aquaticus
<400> PreSequenceString :
atggctaacg aattaacctg gcatgacgtg ctggctgaag agaagcagca accctatttt 60
cttaataccc ttcagaccgt cgccagcgag cggcagtccg gcgtcactat ctacccacca 120
caaaaagatg tctttaacgc gttccgcttt acagagttgg gtgacgttaa agtggtgatt 180
ctcggccagg atccttatca cggaccggga caggcgcatg gtctggcatt ttccgttcgt 240
cccggcattg ccattcctcc gtcattattg aatatgtata aagagctgga aaatactatt 300

ccgggcttca cccgccctaa tcatggttat cttgaaagct gggcgcgtca gggcgttctg 360
ctactcaata ctgtgttgac ggtacgcgca ggtcaggcgc attcccacgc cagcctcggc 420
tgggaaacct tcaccgataa agtgatcagc ctgattaacc agcatcgcga aggcgtggtg 480
tttttgttgt ggggatcgca tgcgcaaaag aaaggggcga ttatagataa gcaacgccat 540
catgtactga aagcaccgca tccgtcgccg ctttcggcgc atcgtggatt ctttggctgc 600
aaccattttg tgctggcaaa tcagtggctg gaacaacgtg gcgagacgcc gattgactgg 660
atgccagtat taccggcaga gagtgagtaa 690
<212> Type : DNA
<211> Length : 690
SequenceName : SEQ ID NO. 9 E. coli Uracil-N-Glycosylase--complete
nucleotide sequence
SequenceDescription :

Sequence
--------
<213> OrganismName : Thermus aquaticus
<400> PreSequenceString :
MANELTWHDV LAEEKQQPYF LNTLQTVASE RQSGVTIYPP QKDVFNAFRF TELGDVKVVI 60
LGQDPYHGPG QAHGLAFSVR PGIAIPPSLL NMYKELENTI PGFTRPNHGY LESWARQGVL 120
LLNTVLTVRA GQAHSHASLG WETFTDKVIS LINQHREGVV FLLWGSHAQK KGAIIDKQRH 180
HVLKAPHPSP LSAHRGFFGC NHFVLANQWL EQRGETPIDW MPVLPAESE 229
<212> Type : PRT
<211> Length : 229
SequenceName : SEQ ID NO. 10 E. coli Uracil-N-Glycosylase --complete amino
acid sequence
SequenceDescription :

SEQUENCE LISTING

<110>  QIAGEN GMBH

<120>  MODIFIED ENZYMES AND THEREIR USES

<130>  N3003 EP BLN

<160>  10

<170>  PatentIn version 3.3

<210>  1
<211>  2499
<212>  DNA
<213>  Thermus aquaticus

<400>  1
atgaggggga tgctgcccct ctttgagccc aagggccggg tcctcctggt ggacggccac      60

cacctggcct accgcacctt ccacgccctg aagggcctca ccaccagccg gggggagccg     120

gtgcaggcgg tctacggctt cgccaagagc ctcctcaagg ccctcaagga ggacggggac     180

gcggtgatcg tggtctttga cgccaaggcc ccctccttcc gccacgaggc ctacgggggg     240

tacaaggcgg gccgggcccc cacgccggag gactttcccc ggcaactcgc cctcatcaag     300

gagctggtgg acctcctggg gctggcgcgc ctcgaggtcc cgggctacga ggcggacgac     360

gtcctggcca gcctggccaa gaaggcggaa aaggagggct acgaggtccg catcctcacc     420

gccgacaaag acctttacca gctcctttcc gaccgcatcc acgccctcca ccccgagggg     480

tacctcatca ccccggcctg gctttgggaa aagtacggcc tgaggcccga ccagtgggcc     540

gactaccggg ccctgaccgg ggacgagtcc gacaaccttc ccggggtcaa gggcatcggg     600

gagaagacgg cgaggaagct tctggaggag tgggggagcc tggaagccct cctcaagaac     660

ctggaccggc tgaagcccgc catccgggag aagatcctgg cccacatgga cgatctgaag     720

ctctcctggg acctggccaa ggtgcgcacc gacctgcccc tggaggtgga cttcgccaaa     780

aggcgggagc ccgaccggga gaggcttagg gcctttctgg agaggcttga gtttggcagc     840

ctcctccacg agttcggcct tctggaaagc cccaaggccc tggaggaggc cccctggccc     900

ccgccggaag gggccttcgt gggctttgtg ctttcccgca aggagcccat gtgggccgat     960

cttctggccc tggccgccgc cagggggggc cgggtccacc gggcccccga gccttataaa    1020

gccctcaggg acctgaagga ggcgcggggg cttctcgcca aagacctgag cgttctggcc    1080

ctgagggaag gccttggcct cccgcccggc gacgacccca tgctcctcgc ctacctcctg    1140

gacccttcca acaccacccc cgagggggtg gcccggcgct acggcgggga gtggacggag    1200

gaggcggggg agcgggccgc cctttccgag aggctcttcg ccaacctgtg ggggaggctt    1260

gagggggagg agaggctcct ttggctttac cgggaggtgg agaggcccct ttccgctgtc    1320

ctggcccaca tggaggccac gggggtgcgc ctggacgtgg cctatctcag ggccttgtcc    1380

ctggaggtgg ccgaggagat cgcccgcctc gaggccgagg tcttccgcct ggccggccac    1440

cccttcaacc tcaactcccg ggaccagctg gaaagggtcc tctttgacga gctagggctt    1500

cccgccatcg gcaagacgga gaagaccggc aagcgctcca ccagcgccgc cgtcctggag 1560
gccctccgcg aggcccaccc catcgtggag aagatcctgc agtaccggga gctcaccaag 1620
ctgaagagca cctacattga ccccttgccg gacctcatcc accccaggac gggccgcctc 1680
cacacccgct tcaaccagac ggccacggcc acgggcaggc taagtagctc cgatcccaac 1740
ctccagaaca tccccgtccg caccccgctt gggcagagga tccgccgggc cttcatcgcc 1800
gaggaggggt ggctattggt ggccctggac tatagccaga tagagctcag ggtgctggcc 1860
cacctctccg gcgacgagaa cctgatccgg gtcttccagg aggggcggga catccacacg 1920
gagaccgcca gctggatgtt cggcgtcccc cgggaggccg tggaccccct gatgcgccgg 1980
gcggccaaga ccatcaactt cggggtcctc tacggcatgt cggcccaccg cctctcccag 2040
gagctagcca tcccttacga ggaggcccag gccttcattg agcgctactt tcagagcttc 2100
cccaaggtgc gggcctggat tgagaagacc ctggaggagg gcaggaggcg ggggtacgtg 2160
gagaccctct tcggccgccg ccgctacgtg ccagacctag aggcccgggt gaagagcgtg 2220
cgggaggcgg ccgagcgcat ggccttcaac atgcccgtcc agggcaccgc cgccgacctc 2280
atgaagctgg ctatggtgaa gctcttcccc aggctggagg aaatgggggc caggatgctc 2340
cttcaggtcc acgacgagct ggtcctcgag gccccaaaag agagggcgga ggccgtggcc 2400
cggctggcca aggaggtcat ggagggggtg tatcccctgg ccgtgcccct ggaggtggag 2460
gtggggatag gggaggactg gctctccgcc aaggagtga 2499

<210> 2
<211> 832
<212> PRT
<213> Thermus aquaticus

<400> 2

Met Arg Gly Met Leu Pro Leu Phe Glu Pro Lys Gly Arg Val Leu Leu
1               5                   10                  15

Val Asp Gly His His Leu Ala Tyr Arg Thr Phe His Ala Leu Lys Gly
            20                  25                  30

Leu Thr Thr Ser Arg Gly Glu Pro Val Gln Ala Val Tyr Gly Phe Ala
        35                  40                  45

Lys Ser Leu Leu Lys Ala Leu Lys Glu Asp Gly Asp Ala Val Ile Val
    50                  55                  60

Val Phe Asp Ala Lys Ala Pro Ser Phe Arg His Glu Ala Tyr Gly Gly
65                  70                  75                  80

Tyr Lys Ala Gly Arg Ala Pro Thr Pro Glu Asp Phe Pro Arg Gln Leu
                85                  90                  95

Ala Leu Ile Lys Glu Leu Val Asp Leu Leu Gly Leu Ala Arg Leu Glu
            100                 105                 110

Val Pro Gly Tyr Glu Ala Asp Asp Val Leu Ala Ser Leu Ala Lys Lys
        115                 120                 125

Ala Glu Lys Glu Gly Tyr Glu Val Arg Ile Leu Thr Ala Asp Lys Asp
    130                 135                 140

Leu Tyr Gln Leu Leu Ser Asp Arg Ile His Ala Leu His Pro Glu Gly
145                 150                 155                 160

Tyr Leu Ile Thr Pro Ala Trp Leu Trp Glu Lys Tyr Gly Leu Arg Pro
                165                 170                 175

Asp Gln Trp Ala Asp Tyr Arg Ala Leu Thr Gly Asp Glu Ser Asp Asn
            180                 185                 190

Leu Pro Gly Val Lys Gly Ile Gly Glu Lys Thr Ala Arg Lys Leu Leu
        195                 200                 205

Glu Glu Trp Gly Ser Leu Glu Ala Leu Leu Lys Asn Leu Asp Arg Leu
    210                 215                 220

Lys Pro Ala Ile Arg Glu Lys Ile Leu Ala His Met Asp Asp Leu Lys
225                 230                 235                 240

Leu Ser Trp Asp Leu Ala Lys Val Arg Thr Asp Leu Pro Leu Glu Val
                245                 250                 255

Asp Phe Ala Lys Arg Arg Glu Pro Asp Arg Glu Arg Leu Arg Ala Phe
            260                 265                 270

Leu Glu Arg Leu Glu Phe Gly Ser Leu Leu His Glu Phe Gly Leu Leu
        275                 280                 285

Glu Ser Pro Lys Ala Leu Glu Glu Ala Pro Trp Pro Pro Pro Glu Gly
    290                 295                 300

Ala Phe Val Gly Phe Val Leu Ser Arg Lys Glu Pro Met Trp Ala Asp
305                 310                 315                 320

Leu Leu Ala Leu Ala Ala Ala Arg Gly Gly Arg Val His Arg Ala Pro
                325                 330                 335

Glu Pro Tyr Lys Ala Leu Arg Asp Leu Lys Glu Ala Arg Gly Leu Leu
            340                 345                 350

Ala Lys Asp Leu Ser Val Leu Ala Leu Arg Glu Gly Leu Gly Leu Pro
        355                 360                 365

Pro Gly Asp Asp Pro Met Leu Leu Ala Tyr Leu Leu Asp Pro Ser Asn
    370                 375                 380

Thr Thr Pro Glu Gly Val Ala Arg Arg Tyr Gly Gly Glu Trp Thr Glu
385                 390                 395                 400

Glu Ala Gly Glu Arg Ala Ala Leu Ser Glu Arg Leu Phe Ala Asn Leu
                405                 410                 415

Trp Gly Arg Leu Glu Gly Glu Glu Arg Leu Leu Trp Leu Tyr Arg Glu
            420                 425                 430

Val Glu Arg Pro Leu Ser Ala Val Leu Ala His Met Glu Ala Thr Gly
        435                 440                 445

Val Arg Leu Asp Val Ala Tyr Leu Arg Ala Leu Ser Leu Glu Val Ala
    450                 455                 460

Glu Glu Ile Ala Arg Leu Glu Ala Glu Val Phe Arg Leu Ala Gly His
465                 470                 475                 480

Pro Phe Asn Leu Asn Ser Arg Asp Gln Leu Glu Arg Val Leu Phe Asp
                485                 490                 495

Glu Leu Gly Leu Pro Ala Ile Gly Lys Thr Glu Lys Thr Gly Lys Arg
            500                 505                 510

Ser Thr Ser Ala Ala Val Leu Glu Ala Leu Arg Glu Ala His Pro Ile
        515                 520                 525

Val Glu Lys Ile Leu Gln Tyr Arg Glu Leu Thr Lys Leu Lys Ser Thr
    530                 535                 540

Tyr Ile Asp Pro Leu Pro Asp Leu Ile His Pro Arg Thr Gly Arg Leu
545                 550                 555                 560

His Thr Arg Phe Asn Gln Thr Ala Thr Ala Thr Gly Arg Leu Ser Ser
                565                 570                 575

Ser Asp Pro Asn Leu Gln Asn Ile Pro Val Arg Thr Pro Leu Gly Gln
            580                 585                 590

Arg Ile Arg Arg Ala Phe Ile Ala Glu Glu Gly Trp Leu Leu Val Ala
        595                 600                 605

Leu Asp Tyr Ser Gln Ile Glu Leu Arg Val Leu Ala His Leu Ser Gly
    610                 615                 620

Asp Glu Asn Leu Ile Arg Val Phe Gln Glu Gly Arg Asp Ile His Thr
625                 630                 635                 640

Glu Thr Ala Ser Trp Met Phe Gly Val Pro Arg Glu Ala Val Asp Pro
                645                 650                 655

Leu Met Arg Arg Ala Ala Lys Thr Ile Asn Phe Gly Val Leu Tyr Gly
            660                 665                 670

Met Ser Ala His Arg Leu Ser Gln Glu Leu Ala Ile Pro Tyr Glu Glu
        675                 680                 685

Ala Gln Ala Phe Ile Glu Arg Tyr Phe Gln Ser Phe Pro Lys Val Arg
    690                 695                 700

Ala Trp Ile Glu Lys Thr Leu Glu Glu Gly Arg Arg Arg Gly Tyr Val
705                 710                 715                 720

Glu Thr Leu Phe Gly Arg Arg Arg Tyr Val Pro Asp Leu Glu Ala Arg
                725                 730                 735

Val Lys Ser Val Arg Glu Ala Ala Glu Arg Met Ala Phe Asn Met Pro
            740                 745                 750

Val Gln Gly Thr Ala Ala Asp Leu Met Lys Leu Ala Met Val Lys Leu
        755                 760                 765

Phe Pro Arg Leu Glu Glu Met Gly Ala Arg Met Leu Leu Gln Val His
    770                 775                 780

Asp Glu Leu Val Leu Glu Ala Pro Lys Glu Arg Ala Glu Ala Val Ala
785                 790                 795                 800

Arg Leu Ala Lys Glu Val Met Glu Gly Val Tyr Pro Leu Ala Val Pro
                805                 810                 815

Leu Glu Val Glu Val Gly Ile Gly Glu Asp Trp Leu Ser Ala Lys Glu
            820                 825                 830

<210>  3
<211>  2503
<212>  DNA
<213>  Thermus aquaticus

<400>  3
atgggccgtg gtatgctgcc gctgtttgaa ccgaaaggcc gtgtgctgct ggttgatggc      60

caccatctgg cctatcgtaa cttttttgcg ctgaaaggcc tgaccacgag ccgtggtgaa     120

ccggtgcagg gcgtgtatgg ctttgcgaaa agcctgctga aagcgctgaa agaggatggc     180

gacgttgtta ttgtggtgtt tgatgcgaaa gcgccgtttt ttcgtcatga agcgtacgaa     240

gcgtataaag cgggccgtgc gccgaccccg gaagattttc cgcgtcagct ggccctgatt     300

aaagagctgg ttgatctgct gggcctggaa cgtctggaag tgccgggctt tgaagcggat     360

gatgtgctgg ccaccctggc caaacaggcg gaacgtgaag gctatgaagt gcgtattctg     420

accgcggatc gtgacctgtt tcagctgctg agcgatcgta ttgcggtgct gcatccggaa     480

ggccatctga ttacgccggg ctggctgtgg gaacgttatg gcctgaaacc ggaacagtgg 540
gtggattttc gtgcgctggc cggcgatccg agcgataaca ttccgggcgt gaaaggcatt 600
ggcgaaaaaa ccgcgctgaa actgctgaaa gaatggggca gcctggaaaa tctgctgaaa 660
aacctggatc atgtgaaacc gccgagcgtg cgtgaaaaaa ttctggccca tctggatgat 720
ctgcgtctgt ctcaggagct gtctcgcgtt cgtaccgatc tgccgctgaa agtggatttt 780
aaaaaacgtc gtgaaccgga tcgtgaaggc ctgaaagcgt ttctggaacg cctggaattt 840
ggcagcctgc tgcatgaatt tggcctgctg gaaagcccgc tgccggcgga agaggcgccg 900
tggccgccac cggaaggtgc gtttctgggc tatcgtctga gccgtccgga accgatgtgg 960
gcggagctgc tggccctggc cgcgagcgcg aaaggtcgtg tgtatcgtgc ggaagaaccg 1020
tatggcgcgc tgcgtggcct gaaagaagtg cgcggcctgc tggctaaaga cctggccgtg 1080
ctggccctgc gtgaaggtct ggatctgccg ccgaccgatg atccgatgct gctggcctat 1140
ctgctggacc cgagcaacac caccccggaa ggtgtggcgc gtcgttatgg cggcgaatgg 1200
accgaagaag cgggcgaacg cgcggttctg agcgaacgtc tgtatgaaaa cctgctgggc 1260
cgtctgcgtg gcgaagaaaa actgctgtgg ctgtatgaag aagtggaaaa accgctgagc 1320
cgtgtgctgg cccatatgga agcgaccggc gtgcgtctgg atgtggcgta tctgaaagcc 1380
ctgagcctgg aagtggcgga agaaatgcgt cgtctggaag aagaagtgtt tcgtctggcc 1440
ggccatccgt ttaacctgaa cagccgtgat cagctggaac gtgtgctgtt tgatgagctg 1500
ggcctgccgc cgattggcaa aaccgaaaaa accggcaaac gtagcaccag cgcggcggtt 1560
ctggaagcgc tgcgtgaagc gcatccgatt gtggaaaaaa tcctgcaata tcgtgagctg 1620
gccaaactga aaggcaccta tattgatccg ctgccggccc tggtgcatcc gaaaaccggc 1680
cgtctgcata cccgttttaa ccagaccgcg accgcgaccg gtcgtctgag cagcagcgat 1740
ccgaacctgc aaaacattcc ggtgcgtacc ccgctgggcc agcgtattcg tcgtgcgttt 1800
gtggccgaag aaggctatct gctggttgcg ctggattaag ccagattgag ctgcgtgttc 1860
tggcccacct gagcggcgat gaaaatctga ttcaggtgtt tcaggaaggc cgcgatattc 1920
atacccagac cgcgagctgg atgtttggcc tgccggccga agcgatcgat ccgctgatgc 1980
gtcgtgcggc gaaaaccatt aactttggcg tgctgtatgg catgagcgcg catcgcctga 2040
gccaggagct gagcattccg tacgaagaag cggtggcgtt tattgatcgt tatttccaga 2100
gctacccgaa agtgaaagcg tggattgaac gtaccctgga agaaggccgt cagcgcggct 2160
atgtggaaac cctgtttggc cgtcgtcgtt atgtgccgga tctgaacgcg cgtgtgaaaa 2220
gcgttcgtga agcggcggaa cgtatggcgt ttaacatgcc ggttcagggc accgcggcgg 2280
atctgatgaa actggcaatg gtgcgtctgt ttccgcgtct gccggaagtg ggtgcgcgta 2340
tgctgctgca agtgcatgat gagctgctgc tggaagcccc gaaagaacgt gcggaagcgg 2400
cggcagccct ggccaaagaa gtgatggagg cgtttggccg ctggccgtgc cgctggaagt 2460
tgaagtgggc attggtgaag attggctgag cgccaaaggc taa 2503

<210> 4
<211> 834
<212> PRT
<213> Thermus aquaticus

<400> 4

Met Gly Arg Gly Met Leu Pro Leu Phe Glu Pro Lys Gly Arg Val Leu
1               5                   10                  15

Leu Val Asp Gly His His Leu Ala Tyr Arg Asn Phe Phe Ala Leu Lys
            20                  25                  30

Gly Leu Thr Thr Ser Arg Gly Glu Pro Val Gln Gly Val Tyr Gly Phe
        35                  40                  45

Ala Lys Ser Leu Leu Lys Ala Leu Lys Glu Asp Gly Asp Val Val Ile
    50                  55                  60

Val Val Phe Asp Ala Lys Ala Pro Phe Phe Arg His Glu Ala Tyr Glu
65                  70                  75                  80

Ala Tyr Lys Ala Gly Arg Ala Pro Thr Pro Glu Asp Phe Pro Arg Gln
                85                  90                  95

Leu Ala Leu Ile Lys Glu Leu Val Asp Leu Leu Gly Leu Glu Arg Leu
            100                 105                 110

Glu Val Pro Gly Phe Glu Ala Asp Asp Val Leu Ala Thr Leu Ala Lys
        115                 120                 125

Gln Ala Glu Arg Glu Gly Tyr Glu Val Arg Ile Leu Thr Ala Asp Arg
    130                 135                 140

Asp Leu Phe Gln Leu Leu Ser Asp Arg Ile Ala Val Leu His Pro Glu
145                 150                 155                 160

Gly His Leu Ile Thr Pro Gly Trp Leu Trp Glu Arg Tyr Gly Leu Lys
                165                 170                 175

Pro Glu Gln Trp Val Asp Phe Arg Ala Leu Ala Gly Asp Pro Ser Asp
            180                 185                 190

Asn Ile Pro Gly Val Lys Gly Ile Gly Glu Lys Thr Ala Leu Lys Leu
        195                 200                 205

Leu Lys Glu Trp Gly Ser Leu Glu Asn Leu Leu Lys Asn Leu Asp His
    210                 215                 220

Val Lys Pro Pro Ser Val Arg Glu Lys Ile Leu Ala His Leu Asp Asp
225                 230                 235                 240

Leu Arg Leu Ser Gln Glu Leu Ser Arg Val Arg Thr Asp Leu Pro Leu
                245                 250                 255

Lys Val Asp Phe Lys Lys Arg Arg Glu Pro Asp Arg Glu Gly Leu Lys
            260                 265                 270

Ala Phe Leu Glu Arg Leu Glu Phe Gly Ser Leu Leu His Glu Phe Gly
        275                 280                 285

Leu Leu Glu Ser Pro Leu Pro Ala Glu Glu Ala Pro Trp Pro Pro Pro
    290                 295                 300

Glu Gly Ala Phe Leu Gly Tyr Arg Leu Ser Arg Pro Glu Pro Met Trp
305                 310                 315                 320

Ala Glu Leu Leu Ala Leu Ala Ala Ser Ala Lys Gly Arg Val Tyr Arg
                325                 330                 335

Ala Glu Glu Pro Tyr Gly Ala Leu Arg Gly Leu Lys Glu Val Arg Gly
            340                 345                 350

Leu Leu Ala Lys Asp Leu Ala Val Leu Ala Leu Arg Glu Gly Leu Asp
        355                 360                 365

Leu Pro Pro Thr Asp Asp Pro Met Leu Leu Ala Tyr Leu Leu Asp Pro
    370                 375                 380

Ser Asn Thr Thr Pro Glu Gly Val Ala Arg Arg Tyr Gly Gly Glu Trp
385                 390                 395                 400

Thr Glu Glu Ala Gly Glu Arg Ala Val Leu Ser Glu Arg Leu Tyr Glu
                405                 410                 415

Asn Leu Leu Gly Arg Leu Arg Gly Glu Glu Lys Leu Leu Trp Leu Tyr
            420                 425                 430

Glu Glu Val Glu Lys Pro Leu Ser Arg Val Leu Ala His Met Glu Ala
        435                 440                 445

Thr Gly Val Arg Leu Asp Val Ala Tyr Leu Lys Ala Leu Ser Leu Glu
    450                 455                 460

Val Ala Glu Glu Met Arg Arg Leu Glu Glu Glu Val Phe Arg Leu Ala
465                 470                 475                 480

Gly His Pro Phe Asn Leu Asn Ser Arg Asp Gln Leu Glu Arg Val Leu
                485                 490                 495

Phe Asp Glu Leu Gly Leu Pro Pro Ile Gly Lys Thr Glu Lys Thr Gly
            500                 505                 510

Lys Arg Ser Thr Ser Ala Ala Val Leu Glu Ala Leu Arg Glu Ala His
        515                 520                 525

Pro Ile Val Glu Lys Ile Leu Gln Tyr Arg Glu Leu Ala Lys Leu Lys
    530                 535                 540

Gly Thr Tyr Ile Asp Pro Leu Pro Ala Leu Val His Pro Lys Thr Gly
545                 550                 555                 560

Arg Leu His Thr Arg Phe Asn Gln Thr Ala Thr Ala Thr Gly Arg Leu
                565                 570                 575

Ser Ser Ser Asp Pro Asn Leu Gln Asn Ile Pro Val Arg Thr Pro Leu
            580                 585                 590

Gly Gln Arg Ile Arg Arg Ala Phe Val Ala Glu Glu Gly Tyr Leu Leu
        595                 600                 605

Val Ala Leu Asp Tyr Ser Gln Ile Glu Leu Arg Val Leu Ala His Leu
    610                 615                 620

Ser Gly Asp Glu Asn Leu Ile Gln Val Phe Gln Glu Gly Arg Asp Ile
625                 630                 635                 640

His Thr Gln Thr Ala Ser Trp Met Phe Gly Leu Pro Ala Glu Ala Ile
                645                 650                 655

Asp Pro Leu Met Arg Arg Ala Ala Lys Thr Ile Asn Phe Gly Val Leu
            660                 665                 670

Tyr Gly Met Ser Ala His Arg Leu Ser Gln Glu Leu Ser Ile Pro Tyr
        675                 680                 685

Glu Glu Ala Val Ala Phe Ile Asp Arg Tyr Phe Gln Ser Tyr Pro Lys
    690                 695                 700

Val Lys Ala Trp Ile Glu Arg Thr Leu Glu Glu Gly Arg Gln Arg Gly
705                 710                 715                 720

Tyr Val Glu Thr Leu Phe Gly Arg Arg Arg Tyr Val Pro Asp Leu Asn
                725                 730                 735

Ala Arg Val Lys Ser Val Arg Glu Ala Ala Glu Arg Met Ala Phe Asn
            740                 745                 750

Met Pro Val Gln Gly Thr Ala Ala Asp Leu Met Lys Leu Ala Met Val
        755                 760                 765

Arg Leu Phe Pro Arg Leu Pro Glu Val Gly Ala Arg Met Leu Leu Gln
    770                 775                 780

Val His Asp Glu Leu Leu Leu Glu Ala Pro Lys Glu Arg Ala Glu Ala
785                 790                 795                 800

Ala Ala Ala Leu Ala Lys Glu Val Met Glu Gly Val Trp Pro Leu Ala
                805                 810                 815

Val Pro Leu Glu Val Glu Val Gly Ile Gly Glu Asp Trp Leu Ser Ala
            820                 825                 830

Lys Gly

<210>  5
<211>  2503
<212>  DNA
<213>  Thermus aquaticus

<400>  5
atgggccgtg gtatgctgcc gctgtttgaa ccgaaaggcc gtgtgctgct ggttgatggc      60

caccatctgg cctatcgtaa cttttttgcg ctgaaaggcc tgaccacgag ccgtggtgaa     120

ccggtgcagg gcgtgtatgg ctttgcgaaa agcctgctga aagcgctgaa agaggatggc     180

gacgttgtta ttgtggtgtt tgatgcgaaa gcgccgtttt ttcgtcatga agcgtacgaa     240

gcgtataaag cgggccgtgc gccgaccccg gaagattttc cgcgtcagct ggccctgatt     300

aaagagctgg ttgatctgct gggcctggaa cgtctggaag tgccgggctt tgaagcggat     360

gatgtgctgg ccaccctggc caaacaggcg gaacgtgaag gctatgaagt gcgtattctg     420

accgcggatc gtgacctgtt tcagctgctg agcgatcgta ttgcggtgct gcatccggaa     480

ggccatctga ttacgccggg ctggctgtgg gaacgttatg gcctgaaacc ggaacagtgg     540

gtggattttc gtgcgctggc cggcgatccg agcgataaca ttccgggcgt gaaaggcatt     600

ggcgaaaaaa ccgcgctgaa actgctgaaa gaatggggca gcctggaaaa tctgctgaaa     660

aacctggatc atgtgaaacc gccgagcgtg cgtgaaaaaa ttctggccca tctggatgat     720

ctgcgtctgt ctcaggagct gtctcgcgtt cgtaccgatc tgccgctgaa agtggatttt     780

aaaaaacgtc gtgaaccgga tcgtgaaggc ctgaaagcgt ttctggaacg cctggaattt     840

ggcagcctgc tgcatgaatt tggcctgctg gaaagcccgc tgccggcgga agaggcgccg     900

tggccgccac cggaaggtgc gtttctgggc tatcgtctga gccgtccgga accgatgtgg     960

gcggagctgc tggccctggc cgcgagcgcg aaaggtcgtg tgtatcgtgc ggaagaaccg    1020

tatggcgcgc tgcgtggcct gaaagaagtg cgcggcctgc tggctaaaga cctggccgtg    1080

ctggccctgc gtgaaggtct ggatctgccg ccgaccgatg atccgatgct gctggcctat    1140

ctgctggacc cgagcaacac caccccggaa ggtgtggcgc gtcgttatgg cggcgaatgg    1200

accgaagaag cgggcgaacg cgcggttctg agcgaacgtc tgtatgaaaa cctgctgggc    1260

cgtctgcgtg gcgaagaaaa actgctgtgg ctgtatgaag aagtggaaaa accgctgagc    1320

cgtgtgctgg cccatatgga agcgaccggc gtgcgtctgg atgtggcgta tctgaaagcc    1380

ctgagcctgg aagtggcgga agaaatgcgt cgtctggaag aagaagtgtt tcgtctggcc 1440

ggccatccgt ttaacctgaa cagccgtgat cagctggaac gtgtgctgtt tgatgagctg 1500

ggcctgccgc cgattggcaa aaccgaaaaa accggcaaac gtagcaccag cgcggcggtt 1560

ctggaagcgc tgcgtgaagc gcatccgatt gtggaaaaaa tcctgcaata tcgtgagctg 1620

gccaaactga aaggcaccta tattgatccg ctgccggccc tggtgcatcc gaaaaccggc 1680

cgtctgcata cccgttttaa ccagaccgcg accgcgaccg gtcgtctgag cagcagcgat 1740

ccgaacctgc aaaacattcc ggtgcgtacc ccgctgggcc agcgtattcg tcgtgcgttt 1800

gtggccgaag aaggctatct gctggttgcg ctggattaag ccagattgag ctgcgtgttc 1860

tggcccacct gagcggcgat gaaaatctga ttcaggtgtt tcaggaaggc cgcgatattc 1920

atacccagac cgcgagctgg atgtttggcc tgccggccga agcgatcgat ccgctgatgc 1980

gtcgtgcggc gaaaaccatt aactttggcg tgctgtatgg catgagcgcg catcgcctga 2040

gccaggagct gagcattccg tacgaagaag cggtggcgtt tattgatcgt tatttccaga 2100

gctacccgaa agtgaaagcg tggattgaac gtaccctgga agaaggccgt cagcgcggct 2160

atgtggaaac cctgtttggc cgtcgtcgtt atgtgccgga tctgaacgcg cgtgtgaaaa 2220

gcgttcgtga agcggcggaa cgtatggcgt ttaacatgcc ggttcagggc accgcggcgg 2280

atctgatgaa actggcaatg gtgcgtctgt ttccgcgtct gccggaagtg ggtgcgcgta 2340

tgctgctgca agtgcatgat gagctgctgc tggaagcccc gaaagaacgt gcggaagcgg 2400

cggcagccct ggccaaagaa gtgatggagg cgtttggccg ctggccgtgc cgctggaagt 2460

tgaagtgggc attggtgaag attggctgag cgccaaatgc taa 2503

<210> 6
<211> 834
<212> PRT
<213> Thermus aquaticus

<400> 6

Met Gly Arg Gly Met Leu Pro Leu Phe Glu Pro Lys Gly Arg Val Leu
1               5                   10                  15

Leu Val Asp Gly His His Leu Ala Tyr Arg Asn Phe Phe Ala Leu Lys
            20                  25                  30

Gly Leu Thr Thr Ser Arg Gly Glu Pro Val Gln Gly Val Tyr Gly Phe
        35                  40                  45

Ala Lys Ser Leu Leu Lys Ala Leu Lys Glu Asp Gly Asp Val Val Ile
    50                  55                  60

Val Val Phe Asp Ala Lys Ala Pro Phe Phe Arg His Glu Ala Tyr Glu
65                  70                  75                  80

Ala Tyr Lys Ala Gly Arg Ala Pro Thr Pro Glu Asp Phe Pro Arg Gln
                85                  90                  95

Leu Ala Leu Ile Lys Glu Leu Val Asp Leu Leu Gly Leu Glu Arg Leu
            100                 105                 110

Glu Val Pro Gly Phe Glu Ala Asp Asp Val Leu Ala Thr Leu Ala Lys
        115                 120                 125

Gln Ala Glu Arg Glu Gly Tyr Glu Val Arg Ile Leu Thr Ala Asp Arg
    130                 135                 140

Asp Leu Phe Gln Leu Leu Ser Asp Arg Ile Ala Val Leu His Pro Glu
145                 150                 155                 160

Gly His Leu Ile Thr Pro Gly Trp Leu Trp Glu Arg Tyr Gly Leu Lys
                165                 170                 175

Pro Glu Gln Trp Val Asp Phe Arg Ala Leu Ala Gly Asp Pro Ser Asp
            180                 185                 190

Asn Ile Pro Gly Val Lys Gly Ile Gly Glu Lys Thr Ala Leu Lys Leu
        195                 200                 205

Leu Lys Glu Trp Gly Ser Leu Glu Asn Leu Leu Lys Asn Leu Asp His
    210                 215                 220

Val Lys Pro Pro Ser Val Arg Glu Lys Ile Leu Ala His Leu Asp Asp
225                 230                 235                 240

Leu Arg Leu Ser Gln Glu Leu Ser Arg Val Arg Thr Asp Leu Pro Leu
                245                 250                 255

Lys Val Asp Phe Lys Lys Arg Arg Glu Pro Asp Arg Glu Gly Leu Lys
            260                 265                 270

Ala Phe Leu Glu Arg Leu Glu Phe Gly Ser Leu Leu His Glu Phe Gly
        275                 280                 285

Leu Leu Glu Ser Pro Leu Pro Ala Glu Glu Ala Pro Trp Pro Pro Pro
    290                 295                 300

Glu Gly Ala Phe Leu Gly Tyr Arg Leu Ser Arg Pro Glu Pro Met Trp
305                 310                 315                 320

Ala Glu Leu Leu Ala Leu Ala Ala Ser Ala Lys Gly Arg Val Tyr Arg
                325                 330                 335

Ala Glu Glu Pro Tyr Gly Ala Leu Arg Gly Leu Lys Glu Val Arg Gly
            340                 345                 350

Leu Leu Ala Lys Asp Leu Ala Val Leu Ala Leu Arg Glu Gly Leu Asp
        355                 360                 365

Leu Pro Pro Thr Asp Asp Pro Met Leu Leu Ala Tyr Leu Leu Asp Pro
    370                 375                 380

Ser Asn Thr Thr Pro Glu Gly Val Ala Arg Arg Tyr Gly Gly Glu Trp
385                 390                 395                 400

Thr Glu Glu Ala Gly Glu Arg Ala Val Leu Ser Glu Arg Leu Tyr Glu
                405                 410                 415

Asn Leu Leu Gly Arg Leu Arg Gly Glu Glu Lys Leu Leu Trp Leu Tyr
            420                 425                 430

Glu Glu Val Glu Lys Pro Leu Ser Arg Val Leu Ala His Met Glu Ala
        435                 440                 445

Thr Gly Val Arg Leu Asp Val Ala Tyr Leu Lys Ala Leu Ser Leu Glu
    450                 455                 460

Val Ala Glu Glu Met Arg Arg Leu Glu Glu Glu Val Phe Arg Leu Ala
465                 470                 475                 480

Gly His Pro Phe Asn Leu Asn Ser Arg Asp Gln Leu Glu Arg Val Leu
                485                 490                 495

Phe Asp Glu Leu Gly Leu Pro Pro Ile Gly Lys Thr Glu Lys Thr Gly
            500                 505                 510

Lys Arg Ser Thr Ser Ala Ala Val Leu Glu Ala Leu Arg Glu Ala His
        515                 520                 525

Pro Ile Val Glu Lys Ile Leu Gln Tyr Arg Glu Leu Ala Lys Leu Lys
    530                 535                 540

Gly Thr Tyr Ile Asp Pro Leu Pro Ala Leu Val His Pro Lys Thr Gly
545                 550                 555                 560

Arg Leu His Thr Arg Phe Asn Gln Thr Ala Thr Ala Thr Gly Arg Leu
                565                 570                 575

Ser Ser Ser Asp Pro Asn Leu Gln Asn Ile Pro Val Arg Thr Pro Leu
            580                 585                 590

Gly Gln Arg Ile Arg Arg Ala Phe Val Ala Glu Glu Gly Tyr Leu Leu
        595                 600                 605

Val Ala Leu Asp Tyr Ser Gln Ile Glu Leu Arg Val Leu Ala His Leu
    610                 615                 620

Ser Gly Asp Glu Asn Leu Ile Gln Val Phe Gln Glu Gly Arg Asp Ile
625                 630                 635                 640

His Thr Gln Thr Ala Ser Trp Met Phe Gly Leu Pro Ala Glu Ala Ile
                645                 650                 655

Asp Pro Leu Met Arg Arg Ala Ala Lys Thr Ile Asn Phe Gly Val Leu
            660                 665                 670

Tyr Gly Met Ser Ala His Arg Leu Ser Gln Glu Leu Ser Ile Pro Tyr
        675                 680                 685

Glu Glu Ala Val Ala Phe Ile Asp Arg Tyr Phe Gln Ser Tyr Pro Lys
    690                 695                 700

Val Lys Ala Trp Ile Glu Arg Thr Leu Glu Glu Gly Arg Gln Arg Gly
705                 710                 715                 720

Tyr Val Glu Thr Leu Phe Gly Arg Arg Arg Tyr Val Pro Asp Leu Asn
                725                 730                 735

Ala Arg Val Lys Ser Val Arg Glu Ala Ala Glu Arg Met Ala Phe Asn
            740                 745                 750

Met Pro Val Gln Gly Thr Ala Ala Asp Leu Met Lys Leu Ala Met Val
        755                 760                 765

Arg Leu Phe Pro Arg Leu Pro Glu Val Gly Ala Arg Met Leu Leu Gln
    770                 775                 780

Val His Asp Glu Leu Leu Leu Glu Ala Pro Lys Glu Arg Ala Glu Ala
785                 790                 795                 800

Ala Ala Ala Leu Ala Lys Glu Val Met Glu Gly Val Trp Pro Leu Ala
                805                 810                 815

Val Pro Leu Glu Val Glu Val Gly Ile Gly Glu Asp Trp Leu Ser Ala
            820                 825                 830

Lys Cys

<210>  7
<211>  2503
<212>  DNA
<213>  Thermus aquaticus

<400>  7
atgggccgtg gtatgctgcc gctgtttgaa ccgaaaggcc gtgtgctgct ggttgatggc      60

caccatctgg cctatcgtaa cttttttgcg ctgaaaggcc tgaccacgag ccgtggtgaa     120

ccggtgcagg gcgtgtatgg ctttgcgaaa agcctgctga aagcgctgaa agaggatggc     180

gacgttgtta ttgtggtgtt tgatgcgaaa gcgccgtttt ttcgtcatga agcgtacgaa     240

gcgtataaag cgggccgtgc gccgaccccg gaagattttc cgcgtcagct ggccctgatt 300
aaagagctgg ttgatctgct gggcctggaa cgtctggaag tgccgggctt tgaagcggat 360
gatgtgctgg ccaccctggc caaacaggcg gaacgtgaag gctatgaagt gcgtattctg 420
accgcggatc gtgacctgtt tcagctgctg agcgatcgta ttgcggtgct gcatccggaa 480
ggccatctga ttacgccggg ctggctgtgg gaacgttatg gcctgaaacc ggaacagtgg 540
gtggattttc gtgcgctggc cggcgatccg agcgataaca ttccgggcgt gaaaggcatt 600
ggcgaaaaaa ccgcgctgaa actgctgaaa gaatggggca gcctggaaaa tctgctgaaa 660
aacctggatc atgtgaaacc gccgagcgtg cgtgaaaaaa ttctggccca tctggatgat 720
ctgcgtctgt ctcaggagct gtctcgcgtt cgtaccgatc tgccgctgaa agtggatttt 780
aaaaaacgtc gtgaaccgga tcgtgaaggc ctgaaagcgt ttctggaacg cctggaattt 840
ggcagcctgc tgcatgaatt tggcctgctg gaaagcccgc tgccggcgga agaggcgccg 900
tggccgccac cggaaggtgc gtttctgggc tatcgtctga gccgtccgga accgatgtgg 960
gcggagctgc tggccctggc cgcgagcgcg aaaggtcgtg tgtatcgtgc ggaagaaccg 1020
tatggcgcgc tgcgtggcct gaaagaagtg cgcggcctgc tggctaaaga cctggccgtg 1080
ctggccctgc gtgaaggtct ggatctgccg ccgaccgatg atccgatgct gctggcctat 1140
ctgctggacc cgagcaacac caccccggaa ggtgtggcgc gtcgttatgg cggcgaatgg 1200
accgaagaag cgggcgaacg cgcggttctg agcgaacgtc tgtatgaaaa cctgctgggc 1260
cgtctgcgtg gcgaagaaaa actgctgtgg ctgtatgaag aagtggaaaa accgctgagc 1320
cgtgtgctgg cccatatgga agcgaccggc gtgcgtctgg atgtggcgta tctgaaagcc 1380
ctgagcctgg aagtggcgga agaaatgcgt cgtctggaag aagaagtgtt tcgtctggcc 1440
ggccatccgt ttaacctgaa cagccgtgat cagctggaac gtgtgctgtt tgatgagctg 1500
ggcctgccgc cgattggcaa aaccgaaaaa accggcaaac gtagcaccag cgcggcggtt 1560
ctggaagcgc tgcgtgaagc gcatccgatt gtggaaaaaa tcctgcaata tcgtgagctg 1620
gccaaactga aaggcaccta tattgatccg ctgccggccc tggtgcatcc gaaaaccggc 1680
cgtctgcata cccgttttaa ccagaccgcg accgcgaccg gtcgtctgag cagcagcgat 1740
ccgaacctgc aaaacattcc ggtgcgtacc ccgctgggcc agcgtattcg tcgtgcgttt 1800
gtggccgaag aaggctatct gctggttgcg ctggattaag ccagattgag ctgcgtgttc 1860
tggcccacct gagcggcgat gaaaatctga ttcaggtgtt tcaggaaggc cgcgatattc 1920
atacccagac cgcgagctgg atgtttggcc tgccggccga agcgatcgat ccgctgatgc 1980
gtcgtgcggc gaaaaccatt aactttggcg tgctgtatgg catgagcgcg catcgcctga 2040
gccaggagct gagcattccg tacgaagaag cggtggcgtt tattgatcgt tatttccaga 2100
gctacccgaa agtgaaagcg tggattgaac gtaccctgga agaaggccgt cagcgcggct 2160
atgtggaaac cctgtttggc cgtcgtcgtt atgtgccgga tctgaacgcg cgtgtgaaaa 2220
gcgttcgtga agcggcggaa cgtatggcgt ttaacatgcc ggttcagggc accgcggcgg 2280

atctgatgaa actggcaatg gtgcgtctgt ttccgcgtct gccggaagtg ggtgcgcgta 2340

tgctgctgca agtgcatgat gagctgctgc tggaagcccc gaaagaacgt gcggaagcgg 2400

cggcagccct ggccaaagaa gtgatggagg cgtttggccg ctggccgtgc cgctggaagt 2460

tgaagtgggc tgtggtgaag attggctgag cgccaaaggc taa 2503

<210> 8
<211> 834
<212> PRT
<213> Thermus aquaticus

<400> 8

Met Gly Arg Gly Met Leu Pro Leu Phe Glu Pro Lys Gly Arg Val Leu
1               5                   10                  15

Leu Val Asp Gly His His Leu Ala Tyr Arg Asn Phe Phe Ala Leu Lys
            20                  25                  30

Gly Leu Thr Thr Ser Arg Gly Glu Pro Val Gln Gly Val Tyr Gly Phe
        35                  40                  45

Ala Lys Ser Leu Leu Lys Ala Leu Lys Glu Asp Gly Asp Val Val Ile
    50                  55                  60

Val Val Phe Asp Ala Lys Ala Pro Phe Phe Arg His Glu Ala Tyr Glu
65                  70                  75                  80

Ala Tyr Lys Ala Gly Arg Ala Pro Thr Pro Glu Asp Phe Pro Arg Gln
                85                  90                  95

Leu Ala Leu Ile Lys Glu Leu Val Asp Leu Leu Gly Leu Glu Arg Leu
            100                 105                 110

Glu Val Pro Gly Phe Glu Ala Asp Asp Val Leu Ala Thr Leu Ala Lys
        115                 120                 125

Gln Ala Glu Arg Glu Gly Tyr Glu Val Arg Ile Leu Thr Ala Asp Arg
    130                 135                 140

Asp Leu Phe Gln Leu Leu Ser Asp Arg Ile Ala Val Leu His Pro Glu
145                 150                 155                 160

Gly His Leu Ile Thr Pro Gly Trp Leu Trp Glu Arg Tyr Gly Leu Lys
                165                 170                 175

Pro Glu Gln Trp Val Asp Phe Arg Ala Leu Ala Gly Asp Pro Ser Asp
            180                 185                 190

Asn Ile Pro Gly Val Lys Gly Ile Gly Glu Lys Thr Ala Leu Lys Leu
        195                 200                 205

Leu Lys Glu Trp Gly Ser Leu Glu Asn Leu Leu Lys Asn Leu Asp His
    210                 215                 220

Val Lys Pro Pro Ser Val Arg Glu Lys Ile Leu Ala His Leu Asp Asp
225                 230                 235                 240

Leu Arg Leu Ser Gln Glu Leu Ser Arg Val Arg Thr Asp Leu Pro Leu
                245                 250                 255

Lys Val Asp Phe Lys Lys Arg Arg Glu Pro Asp Arg Glu Gly Leu Lys
            260                 265                 270

Ala Phe Leu Glu Arg Leu Glu Phe Gly Ser Leu Leu His Glu Phe Gly
        275                 280                 285

Leu Leu Glu Ser Pro Leu Pro Ala Glu Glu Ala Pro Trp Pro Pro Pro
    290                 295                 300

Glu Gly Ala Phe Leu Gly Tyr Arg Leu Ser Arg Pro Glu Pro Met Trp
305                 310                 315                 320

Ala Glu Leu Leu Ala Leu Ala Ala Ser Ala Lys Gly Arg Val Tyr Arg
                325                 330                 335

Ala Glu Glu Pro Tyr Gly Ala Leu Arg Gly Leu Lys Glu Val Arg Gly
            340                 345                 350

Leu Leu Ala Lys Asp Leu Ala Val Leu Ala Leu Arg Glu Gly Leu Asp
        355                 360                 365

Leu Pro Pro Thr Asp Asp Pro Met Leu Leu Ala Tyr Leu Leu Asp Pro
    370                 375                 380

Ser Asn Thr Thr Pro Glu Gly Val Ala Arg Arg Tyr Gly Gly Glu Trp
385                 390                 395                 400

Thr Glu Glu Ala Gly Glu Arg Ala Val Leu Ser Glu Arg Leu Tyr Glu
                405                 410                 415

Asn Leu Leu Gly Arg Leu Arg Gly Glu Glu Lys Leu Leu Trp Leu Tyr
            420                 425                 430

Glu Glu Val Glu Lys Pro Leu Ser Arg Val Leu Ala His Met Glu Ala
        435                 440                 445

Thr Gly Val Arg Leu Asp Val Ala Tyr Leu Lys Ala Leu Ser Leu Glu
    450                 455                 460

Val Ala Glu Glu Met Arg Arg Leu Glu Glu Glu Val Phe Arg Leu Ala
465                 470                 475                 480

Gly His Pro Phe Asn Leu Asn Ser Arg Asp Gln Leu Glu Arg Val Leu
                485                 490                 495

Phe Asp Glu Leu Gly Leu Pro Pro Ile Gly Lys Thr Glu Lys Thr Gly
            500                 505                 510

Lys Arg Ser Thr Ser Ala Ala Val Leu Glu Ala Leu Arg Glu Ala His
        515                 520                 525

Pro Ile Val Glu Lys Ile Leu Gln Tyr Arg Glu Leu Ala Lys Leu Lys
    530                 535                 540

Gly Thr Tyr Ile Asp Pro Leu Pro Ala Leu Val His Pro Lys Thr Gly
545                 550                 555                 560

Arg Leu His Thr Arg Phe Asn Gln Thr Ala Thr Ala Thr Gly Arg Leu
                565                 570                 575

Ser Ser Ser Asp Pro Asn Leu Gln Asn Ile Pro Val Arg Thr Pro Leu
            580                 585                 590

Gly Gln Arg Ile Arg Arg Ala Phe Val Ala Glu Glu Gly Tyr Leu Leu
        595                 600                 605

Val Ala Leu Asp Tyr Ser Gln Ile Glu Leu Arg Val Leu Ala His Leu
    610                 615                 620

Ser Gly Asp Glu Asn Leu Ile Gln Val Phe Gln Glu Gly Arg Asp Ile
625                 630                 635                 640

His Thr Gln Thr Ala Ser Trp Met Phe Gly Leu Pro Ala Glu Ala Ile
                645                 650                 655

Asp Pro Leu Met Arg Arg Ala Ala Lys Thr Ile Asn Phe Gly Val Leu
            660                 665                 670

Tyr Gly Met Ser Ala His Arg Leu Ser Gln Glu Leu Ser Ile Pro Tyr
        675                 680                 685

Glu Glu Ala Val Ala Phe Ile Asp Arg Tyr Phe Gln Ser Tyr Pro Lys
    690                 695                 700

Val Lys Ala Trp Ile Glu Arg Thr Leu Glu Glu Gly Arg Gln Arg Gly
705                 710                 715                 720

Tyr Val Glu Thr Leu Phe Gly Arg Arg Arg Tyr Val Pro Asp Leu Asn
                725                 730                 735

Ala Arg Val Lys Ser Val Arg Glu Ala Ala Glu Arg Met Ala Phe Asn
            740                 745                 750

Met Pro Val Gln Gly Thr Ala Ala Asp Leu Met Lys Leu Ala Met Val
        755                 760                 765

Arg Leu Phe Pro Arg Leu Pro Glu Val Gly Ala Arg Met Leu Leu Gln
    770                 775                 780

Val His Asp Glu Leu Leu Leu Glu Ala Pro Lys Glu Arg Ala Glu Ala
785                 790                 795                 800

Ala Ala Ala Leu Ala Lys Glu Val Met Glu Gly Val Trp Pro Leu Ala
                805                 810                 815

Val Pro Leu Glu Val Glu Val Gly Cys Gly Glu Asp Trp Leu Ser Ala
            820                 825                 830

Lys Gly

<210>  9
<211>  690
<212>  DNA
<213>  Thermus aquaticus

<400>  9
atggctaacg aattaacctg gcatgacgtg ctggctgaag agaagcagca accctatttt      60

cttaataccc ttcagaccgt cgccagcgag cggcagtccg gcgtcactat ctacccacca     120

caaaaagatg tctttaacgc gttccgcttt acagagttgg gtgacgttaa agtggtgatt     180

ctcggccagg atccttatca cggaccggga caggcgcatg gtctggcatt ttccgttcgt     240

cccggcattg ccattcctcc gtcattattg aatatgtata aagagctgga aaatactatt     300

ccgggcttca cccgccctaa tcatggttat cttgaaagct gggcgcgtca gggcgttctg     360

ctactcaata ctgtgttgac ggtacgcgca ggtcaggcgc attcccacgc cagcctcggc     420

tgggaaacct tcaccgataa agtgatcagc ctgattaacc agcatcgcga aggcgtggtg     480

tttttgttgt ggggatcgca tgcgcaaaag aaaggggcga ttatagataa gcaacgccat     540

catgtactga aagcaccgca tccgtcgccg ctttcggcgc atcgtggatt ctttggctgc     600

aaccattttg tgctggcaaa tcagtggctg gaacaacgtg gcgagacgcc gattgactgg     660

atgccagtat taccggcaga gagtgagtaa                                      690

<210>  10
<211>  229
<212>  PRT
<213>  Thermus aquaticus

<400>  10

Met Ala Asn Glu Leu Thr Trp His Asp Val Leu Ala Glu Glu Lys Gln
1               5                   10                  15

Gln Pro Tyr Phe Leu Asn Thr Leu Gln Thr Val Ala Ser Glu Arg Gln
            20                  25                  30

Ser Gly Val Thr Ile Tyr Pro Pro Gln Lys Asp Val Phe Asn Ala Phe
        35                  40                  45

Arg Phe Thr Glu Leu Gly Asp Val Lys Val Val Ile Leu Gly Gln Asp
    50                  55                  60

Pro Tyr His Gly Pro Gly Gln Ala His Gly Leu Ala Phe Ser Val Arg
65                  70                  75                  80

Pro Gly Ile Ala Ile Pro Pro Ser Leu Leu Asn Met Tyr Lys Glu Leu
                85                  90                  95

Glu Asn Thr Ile Pro Gly Phe Thr Arg Pro Asn His Gly Tyr Leu Glu
            100                 105                 110

Ser Trp Ala Arg Gln Gly Val Leu Leu Leu Asn Thr Val Leu Thr Val
        115                 120                 125

Arg Ala Gly Gln Ala His Ser His Ala Ser Leu Gly Trp Glu Thr Phe
    130                 135                 140

Thr Asp Lys Val Ile Ser Leu Ile Asn Gln His Arg Glu Gly Val Val
145                 150                 155                 160

Phe Leu Leu Trp Gly Ser His Ala Gln Lys Lys Gly Ala Ile Ile Asp
                165                 170                 175

Lys Gln Arg His His Val Leu Lys Ala Pro His Pro Ser Pro Leu Ser
            180                 185                 190

Ala His Arg Gly Phe Phe Gly Cys Asn His Phe Val Leu Ala Asn Gln
        195                 200                 205

Trp Leu Glu Gln Arg Gly Glu Thr Pro Ile Asp Trp Met Pro Val Leu
    210                 215                 220

Pro Ala Glu Ser Glu
225

## Claims

1. Enzyme of bacterial, fungal, viral, or archae origin, wherein the enzyme is coupled with at least one polymer having a molecular weight between about 500 and 50,000 daltons selected from the group consisting of polyethylene glycol and polypropylene glycol.

2. The enzyme of claim 1, wherein the polymer has a molecular weight of between about 750 and 20,000 daltons.

3. The enzyme of claims 1 to 2, wherein the enzyme carries between 1 and 100 polymer moieties per enzyme molecule.

4. The enzyme of claims 1 to 3, wherein the polymer is polyethylene glycol.

5. The enzyme of claims 1 to 4, wherein the polymer has at least one functional endgroup, or one homobifunctional endgroup or one heterobifunctional endgroup.

6. The enzyme of claims 1 to 5, wherein the polymer has at least one heterobifunctional endgroup selected from the group of maleimide, vinyl sulphones, pyridyl disulphide, amine, carboxylic acids and NHS esters.

7. The enzyme of claims 1 to 6, wherein the polymer has at least one endgroup which is maleimide.

8. The enzyme of claims 1 to 7, wherein the polymer is bound to a reactive amino acid selected from the group of lysine, cysteine, histidine, arginine, aspartic acid, glutamic acid, serine, threonine and tyrosine.

9. The enzyme of claims 1 to 8, wherein the polymer is bound to a reactive amino acid located close to the N-terminal end of the enzyme or close to the C-terminal end of the enzyme.

10. The enzyme of claim 9, wherein the polymer is bound to the N-terminal amino group and/or the C-terminal carboxylic acid.

11. The enzyme of claim 1 to 10, wherein the enzyme is selected from the group of fungal alpha-amylase, protease, trypsin, amylase, glucanase, protease, betaglucanase, arabinoxylanases, amyloglucosidase, pullulanases, acetolactatedecarboxylase (ALDC), pectinases, rennin, lipases, lactases papain, glucoamylases, glucose isomerase, xylanase, cellulase, ligninase, restriction enzymes, DNA ligase, polymerases, , a ligase, an endonuclease, an exonuclease, methyltransferase, recombinase, polynucleotide kinase, phosphatases sulfurylases, and Uracil-DNA N-glycasylase.

12. The enzyme of claim 1, wherein the enzyme carries at least a nucleic acid modifying or replicating activity.

13. The enzyme of claims 1 to 12, wherein the enzyme's substrate is a nucleic acid.

14. The enzyme of claim 13, wherein the enzyme is selected from the group of, polymerase, ligase, endonuclease, exonuclease, methyltransferase, recombinase, polynucleotide kinase, phosphatases, sulfurylases.

15. The enzyme according to claim 14, wherein the polymerase is a DNA-dependent DNA polymerase, an RNA-dependent DNA polymerase, RNA-dependent RNA polymerase.

16. The enzyme according to claim 15, wherein the polymerase is from a thermophilic organism.

17. The enzyme according to claim 16, wherein the polymerase is selected from the group of genii of *Thermus, Aquifex, Thermotoga, Thermocridis, Hydrogenobacter, Thermosynchecoccus* and *Thermoanaerobacter.*

18. The enzyme according to claim 17, wherein the polymerase is selected from the group of organisms of *Aquifex aeolicus, Aquifex pyogenes, Thermus thermophilus, Therms aquaticus, Thermotoga neapolitana, Thermus pacificus* and *Thermotoga maritima.*

19. The enzyme according to claims 15 to 18, wherein the enzyme is a Pol-A-type polymerase.

20. The enzyme according to any of the above claims, wherein the at least one polymer is bound to an amino acid that is not present in the amino acid sequence of the enzyme in its natural state but has been incorporated into the enzyme either in addition to an existing amino acid or at the position of an existing amino acid.

21. The enzyme according to claim 20, wherein the at least one polymer is coupled to an amino acid selected from the group of lysine and cysteine.

22. The enzyme according to claims 20 to 21, wherein the amino acid is surface exposed.

23. Polymerase according to any of the above claims 15 to 22, wherein the at least one polymer is coupled to one or more cysteine and/or lysine residues that have been incorporated into an A-type polymerase at a position corresponding to the position of the following amino acids of Taq polymerase Leu⁴⁶¹, Ala⁵²¹, Gly⁶⁴⁸, Ala⁶⁵³, Ser⁶⁷⁹, Ala⁶⁸³, Ser⁶⁹⁹, Ser⁷³⁹ Ala⁸¹⁴, Ser⁸²⁹ and Glu⁸³²_{.}

24. Method for modifying or amplifying a nucleic acid, wherein the nucleic acid to be modified or amplified is present in a reaction mixture comprising an enzyme according to any of the claims 12 to 23.

25. Method according to claim 24, wherein the reaction is an amplification reaction and the enzyme is a polymerase.
